# EUROPEAN PATENT APPLICATION

(11) **EP 0 962 532 A1**
(43) Date of publication of application: **08.12.1999**
(21) Application number: 98870127.2
(22) Date of filing: 03.06.1998
(51) Int. Cl.: C12N 15/31, C07K 14/35, C07K 19/00, C07K 16/12, G01N 33/569

(54) **Polypeptides and nucleic acids encoding the same for the diagnosis and prevention of tuberculosis disease.**

(71) Applicant: INNOGENETICS N.V., 9052 Gent (BE)
(72) Inventor: Cocito, Carlo, 1340 Ottignies (BE); Coetsier, Christophe, 5530 Spontin (BE)

(57) **Abstract**

The invention relates to an isolated nucleic acid of *M. tuberculosis* containing in its nucleotide sequence at least two overlapping ORF's which encode the following two antigenic proteins, recognized by a pool of sera from tuberculous patients:
(i) B2 protein represented by SEQ ID N0 2, or a protein homologous to B2 containing in its amino acid sequence at least one conservative amino acid substitution (according to Table 1) with respect to the sequence represented by SEQ ID N0 2, and
(ii) B3 protein represented by SEQ ID N0 3, or a protein homologous to B3 containing in its amino acid sequence at least one conservative amino acid substitution (according to Table 1) with respect to the sequence represented by SEQ ID N0 3,
   or a fragment of said nucleic acid, said fragment containing at least 15, preferably at least 30 and more preferably at least 45 contiguous nucleotides of the sequence of said nucleic acid, and encoding at least one epitope of the B2 and/or B3 protein, or its homologues.

## Description

The current invention relates to an isolated nucleic acid molecule of *M. tuberculosis*, encoding several antigenic polypeptides useful for the *in vitro* diagnosis and prevention of tuberculosis disease in mammals. The invention also relates to recombinant polypeptides encoded by said nucleic acid, methods for producing the same, and their use in methods and kits for the *in vitro* diagnosis of tuberculosis disease in mammals. The invention also relates to a vaccine for the prevention of tuberculosis disease in mammals, comprising as an active principle said nucleic acid or the polypeptides encoded thereby.

### BACKGROUND OF THE INVENTION

Tuberculosis is the infectious disease with the largest diffusion worldwide, affecting about one third of the world population. Its high morbidity (8 million new patients a year) and mortality (3 million deaths a year) rates witness the virulence of the etiological agents (*M. tuberculosis* and *M. bovis*).

Tuberculous bacilli are disseminated by post-primary tuberculosis patients with open lesions (Daniel, 1994; Youmans, 1985). The commonly used diagnostic procedures are unsatisfactory. Culturing the bacilli starting from a sample taken from the patient requires very long incubation times, while only mycobacteria with intact envelop are identified in biopsies by the Ziehl-Neelsen staining. These handicaps are not shared by the immunological diagnostic procedures (Daniel and Debanne, 1987). However, commercially available serological methods lack species-specificity, being unable to distinguish tuberculous bacilli from the other pathogenic and saprophytic mycobacteria. In fact, extensive homologies exist among the corresponding proteins of different mycobacterial species.

Numerous proteins of *M.tuberculosis* and *M.bovis* have been identified as immunologically significant for diagnosis, being recognized by patients sera and/or eliciting a cellular immune response. Most of them are secretory products, released by growing bacteria and thus directly interacting with the host immune system. Reference can be made to the review article of Young et al (1992) for the mycobacterial proteins described before 1992. Those more recently discovered have molecular weights of 6 (Sörensen et al. 1995; Pollock and Andersen, 1997), 10-14 (Boom et al., 1994), 16 (Verbon et al. 1992; Friscia et al., 1995), 18 (Manca et al. 1997), 19 (Harris et al. 1994, Prestidge et al. 1995), 25 (Hewinson et al. 1996), 26 (O'Loan et al., 1994), 28 (Berthet et al., 1995; Bigi et al. 1995), 29 (Laqueyrerie et al., 1995), 30 (Sinha et al., 1997), 34 (Hermans et al., 1995), 45 (Romain et al., 1993), 66 (Deshpande et al., 1994) and 88 (Braibant et al., 1994; Laal et al., 1997) kDa.

A small number of mycobacterial protein genes have been cloned: the corresponding translation products have been characterized, and some of them have been used on an experimental scale for immunoassays. The heat-shock proteins of 14, 65, and 71 kDa of the tuberculous bacilli have been purified (Young et al. 1992): they cross-react, however, with the homologous proteins of other species, since they hold ancient motifs shared by even distantly-related bacteria (Young et al. 1987). Among the recombinant proteins biochemically characterized, those of 6 kDa (ESAT-6) (Sörensen et al., 1995), 18 kDa (MPT-63) (Manca et al., 1997), 19 kDa (Prestidge et al., 1995), 22 and 25 kDa (MPB83 and 70) (Hewinson et al., 1996; Matsumoto et al., 1995), 28 kDa (erp) (Berthet et al., 1995; Bigi et al., 1995), 29 kDa (Apa 45/47) (Laqueyrerie et al., 1995), 30-32 kDa (Ag 85 A-B-C) (Borremans et al., 1989; Content et al., 1991), 34 kDa (Ag 84) (Hermans et al., 1995), 38 kDa (Singh et al., 1992), ought to be cited. Most of these proteins crossreact with the corresponding proteins of mycobacteria other than *M. tuberculosis*. None of them has been used so far as a reagent in commercially available diagnostic kits, or applied for large scale epidemiological investigation. Detection assays based on these antigens still lack sensitivity and/or specificity. In order to enhance sensitivity, a combination of different *M. tuberculosis* antigens will be indispensable. Specificity of the assay has to be increased by selecting peptides which specifically react with sera from *M. tuberculosis* (*M. bovis*) infected subjects and not with sera from healthy subjects, or subjects infected by mycobacteria other than *M. tuberculosis*. There remains a continuing need for new *M. tuberculosis* antigens, more particularly antigens containing *M. tuberculosis* species-specific epitopes useful in the immunodiagnosis of tuberculosis.

### AIMS

It is an aim of the current invention to provide new *M. tuberculosis* antigens useful in the immunodiagnosis of tuberculosis disease.

More specifically, it is an aim of the current invention to provide new *M. tuberculosis* polypeptides containing species-specific epitopes, useful in the serodiagnosis of tuberculosis disease.

It is moreover an aim of the current invention to provide the nucleic acids encoding said polypeptides. The sequence of said nucleic acids and their translation products is also provided by the current invention.

It is also an aim of the current invention to provide methods for producing said *M. tuberculosis* polypeptides.

It is moreover an aim of the current invention to provide methods and kits for immunodiagnosis of tuberculosis disease in mammals.

Finally, it is an aim of the current invention to provide for a vaccine composition against tuberculosis disease.

It is believed that all the above-mentioned aims have been met by the different embodiments of the current invention.

### DEFINITIONS

The term "nucleic acid" or "polynucleic acid" refers to a single stranded or double stranded nucleic acid molecule which may contain from 10 nucleotides to the complete nucleotide sequence (such as for instance 20, 30, 40, 50, 60, 70, 80 or more nucleotides). A nucleic acid which is smaller than about 100 nucleotides in length is often also referred to as an oligonucleotide. A nucleic acid may consist of deoxyribonucleotides (DNA) or ribonucleotides (RNA), nucleotide analogues or modified nucleotides, or may have been adapted for therapeutic purposes. Nucleic acid molecules are represented throughout the current invention by single stranded DNA-sequences, extending from the 5' end to the 3' end. It should be understood however that the nucleic acids of the current invention also encompass the complementary strand of the illustrated molecule, as well as the double stranded molecules and the corresponding RNA molecules, wherein the deoxyribonucleotides are replaced by ribonucleotides, and wherein T is replaced by U.

The expression "isolated" means that the respective component (nucleic acid, protein) is preferably 90%, more preferably 95%, most preferably 98% pure as measured by its weight versus the weight of possible contaminants. For the isolated polypeptides of the invention, the purity is high enough in order to ensure a specific reactivity of the polypeptides with sera from tuberculous patients.

The term "Open Reading Frame (ORF)" refers to a nucleotide sequence containing a series of triplets coding for amino acids, without the occurrence of a termination codon. The sequence of an ORF is (potentially) translatable into a protein.

The term "polypeptide" designates a linear series of amino acids connected one to the other by peptide bonds between the alpha-amino and carboxy groups of adjacent amino acids. In the context of the present invention, the term "polypeptide" may be used interchangeably with the term "protein". Polypeptides can be in a variety of lengths, shorter polypeptides (i.e. of 50 amino acids in length or shorter), are usually termed "peptides". Polypeptides and peptides may occur either in their natural (uncharged) forms or in forms which are salts, and either free of modifications such as glycosylation, side chain oxidation, or phosphorylation or containing these modifications. It is well understood in the art that amino acid sequences contain acidic and basic groups, and that the particular ionization state exhibited by the polypeptide is dependent on the pH of the surrounding medium when the protein is in solution, or that of the medium from which it was obtained if the protein is in solid form. Also included in the definition are proteins modified by additional substituents attached to the amino acids side chains, such as glycosyl units, lipids, or inorganic ions such as phosphates, as well as modifications relating to chemical conversions of the chains, such as oxidation of sulfhydryl groups. Thus the polypeptides of the invention also include the appropriate amino acid sequence referenced subject to those of the foregoing modifications which do not destroy its functionality (i.e. immunoreactivity).

The polypeptides of the invention, and particularly the shorter peptides, can be prepared by classical chemical synthesis. The synthesis can be carried out in homogeneous solution or on solid phase.

For instance, the synthesis technique in homogeneous solution which can be used is the one described by Houbenweyl in the book entitled "Methode der organischen chemie" (Method of organic chemistry) edited by E. Wunsh, vol. 15-I et II. THIEME, Stuttgart 1974.

The polypeptides of the invention can also be prepared in solid phase according to the methods described by Atherton and Shepard in their book entitled "Solid phase peptide synthesis" (IRL Press, Oxford, 1989).

The polypeptides according to this invention can also be prepared by means of recombinant DNA techniques as described by Maniatis et al., (Molecular Cloning: A Laboratory Manual, New York, Cold Spring Harbor Laboratory, 1982), and as illustrated in the examples section.

The term "antigenic polypeptide" means that the polypeptide contains antigenic regions which induce a humoral (i.e. formation of antibodies) or a cellular immune response in the host organism which has been infected by the pathogen from which the polypeptide is isolated.

The term "immunogenic polypeptide" means that the polypeptide induces a protective immune response in the host organism. Immunogenic (poly)peptides are preferred reagents for vaccine compositions.

The term "immunodiagnosis" refers to the diagnosis of a disease by detection of a disease-specific immune response of the host organism. The immune response may be either of the humoral type (raise in antibody titer) or of the cellular type (specific lymphoproliferation, secretion of certain lymphokines...).

The term "serodiagnosis" refers to the diagnosis of a disease by the detection in the serum of a raised titer of specific antibodies against the pathogen.

The term "sensitivity" of an assay refers to the proportion of correctly identified patients. It is calculated as the ratio of "true positives" over the sum of "true positives" and "false negatives".

The term "specificity" of an assay refers to the proportion of healthy (=non-tuberculous) subjects that are serologically negative. It is calculated as the ratio of "true negatives" over the sum of "true negatives" and "false positives".

The term "epitope" or "antigenic determinant" refers to that portion of a molecule (in this case protein molecule) that is specifically bound by an antibody combining site. Epitopes may be determined by any of the techniques known in the art. They may be roughly predicted by a variety of computer prediction models known in the art. An epitope contains usually a stretch of at least 6 amino acids, preferably 7, 8, 9, 10, 11, 12, 13, 14, 15, 16, 17, 18, 19, 20, 21, 22, 23, 24, 25, 30, 50 contiguous amino acids.

The expression "species-specific epitope" refers to an epitope which will only be recognized by antibodies raised against the respective species (organism). For example, a *M. tuberculosis* specific epitope will only be recognized by antibodies raised against the species *M. tuberculosis*, and not by antibodies raised against other types of mycobacteria. Species specificity may be evaluated using antibodies raised *in vitro* in laboratory animals, such as the technique used in the examples section (absorption technique). It may also be evaluated using antibodies present in sera from subjects who have been infected with the respective species.

It should be understood that in the context of the present invention the closely related species *M. tuberculosis* and *M. bovis* have to be considered as one single species, causing tuberculosis in mammals, and more particularly in man and bovine. It should be clear that, where the term *M. tuberculosis* is used, it encompasses in fact all the species belonging to the *M. tuberculosis* complex, and thus includes at least the following species: *M. tuberculosis, M. bovis, M. africanum*.

The expression "immunodominant epitope" refers to an epitope which is recognized by the majority of host immune systems. It means that, if a random set of sera from tuberculous patients is analysed, at least 30%, preferably 40%, and more preferably 50% or more of the sera recognize that particular epitope.

The expression "conservative amino acid substitution" means the substitution of an amino acid by a "related" amino acid, of which the side chain characteristics are comparable to the original amino acid. Table 1 shows a list of which substitutions are generally considered to be conservative.

**TABLE 1**

| **Overview of conservative amino acid substitutions.** | |
|---|---|
| **Amino acid** | **Conservative substitution by** |
| Ser (S) | Thr, Gly, Asn |
| Arg (R) | His, Lys, Glu, Gln |
| Leu (L) | Ile, Met, Phe, Val, Tyr |
| Pro (P) | Ala, Thr, Gly |
| Thr (T) | Pro, Ser, Ala, Gly, His, Gln |
| Ala (A) | Pro, Gly, Thr |
| Val (V) | Met, Ile, Tyr, Phe, Leu |
| Gly (G) | Ala, Thr, Pro, Ser |
| Ile (I) | Met, Leu, Phe, Val, Tyr |
| Phe (F) | Met, Tyr, Ile, Leu, Trp, Val |
| Tyr (Y) | Phe, Trp, Met, Ile, Val, Leu |
| Cys (C) | Ser, Thr, Met |
| His (H) | Gln, Arg, Lys, Glu, Thr |
| Gln (Q) | Glu, His, Lys, Asn, Thr, Arg |
| Asn (N) | Asp, Ser, Gln |
| Lys (K) | Arg, Glu, Gln, His |
| Asp (D) | Asn, Glu, Gln |
| Glu (E) | Gln, Asp, Lys, Asn, His, Arg |
| Met (M) | Ile, Leu, Phe, Val |

### DETAILED DESCRIPTION OF THE INVENTION

The current invention provides for an isolated nucleic acid of *M. tuberculosis* containing a nucleotide sequence with a maximum length of 3000 contiguous nucleotides, wherein said nucleotide sequence contains at least two overlapping ORF's which encode at least a fragment of the following two antigenic proteins recognized by a pool of sera from tuberculous patients:
(i) a B2 protein represented by SEQ ID N0 2, or a protein homologous to B2 containing in its amino acid sequence at least one conservative amino acid substitution (according to Table 1) with respect to the sequence represented by SEQ ID N0 2, and
(ii) a B3 protein represented by SEQ ID N0 3, or a protein homologous to B3 containing in its amino acid sequence at least one conservative amino acid substitution (according to Table 1) with respect to the sequence represented by SEQ ID N0 3,
   or a fragment of said nucleic acid, said fragment containing at least 15, preferably at least 30 and more preferably at least 45 contiguous nucleotides of the sequence of said nucleic acid, and encoding at least one epitope of the B2 and/or B3 protein, or its homologues.

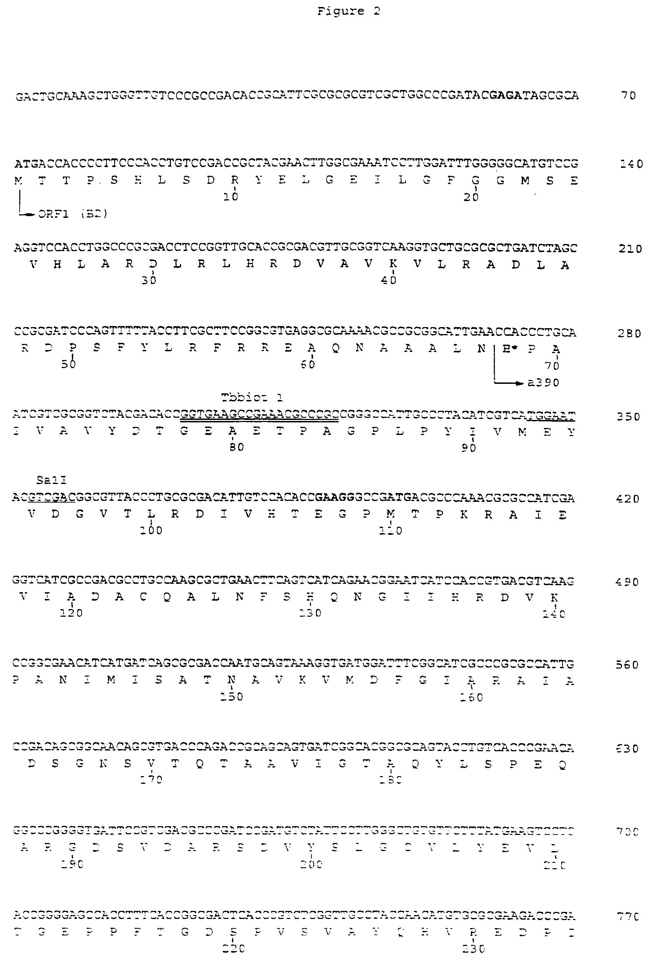

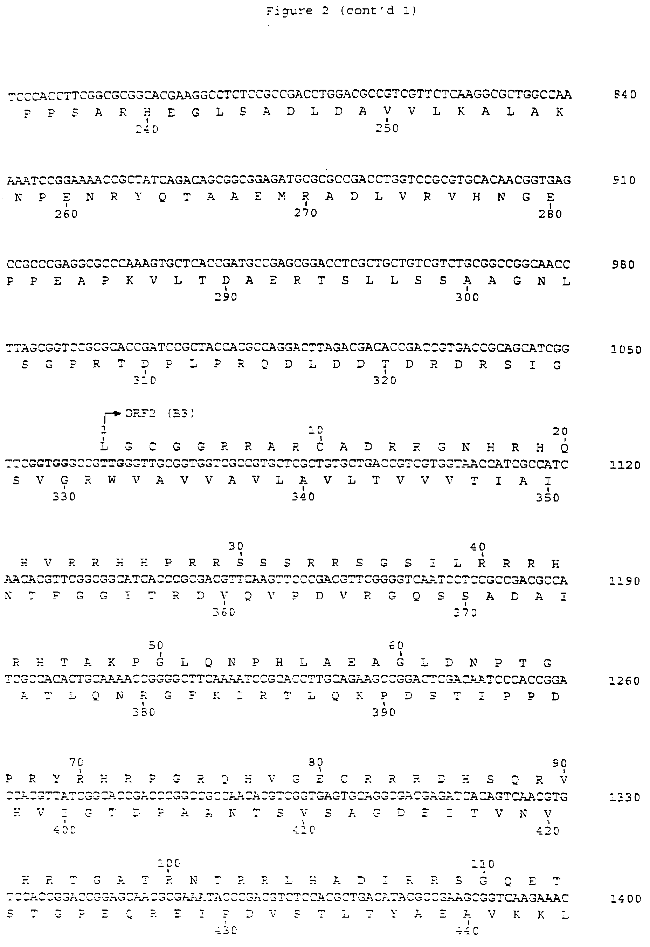

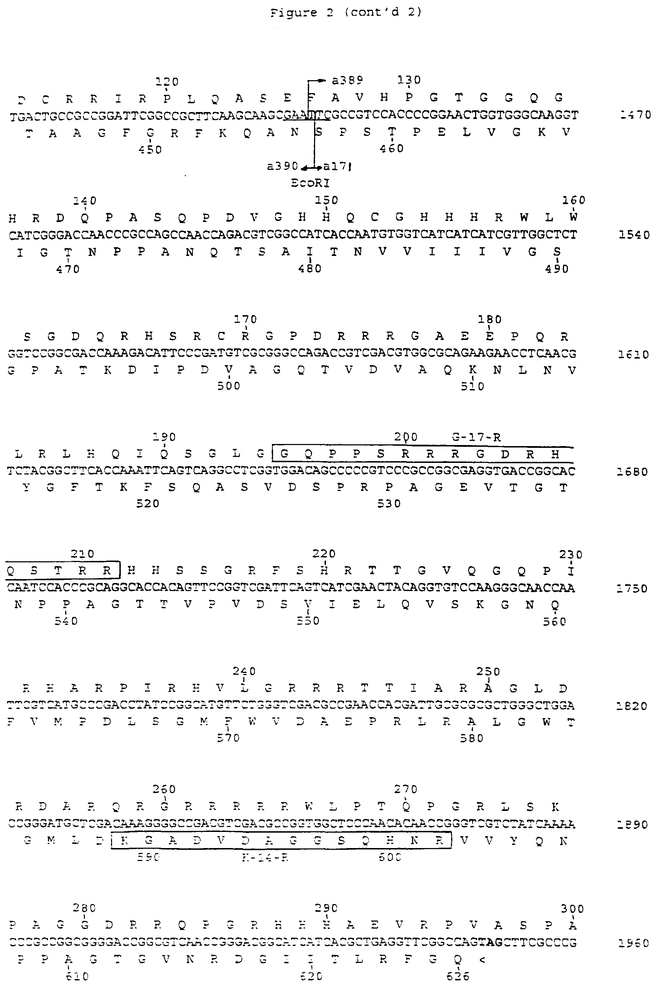

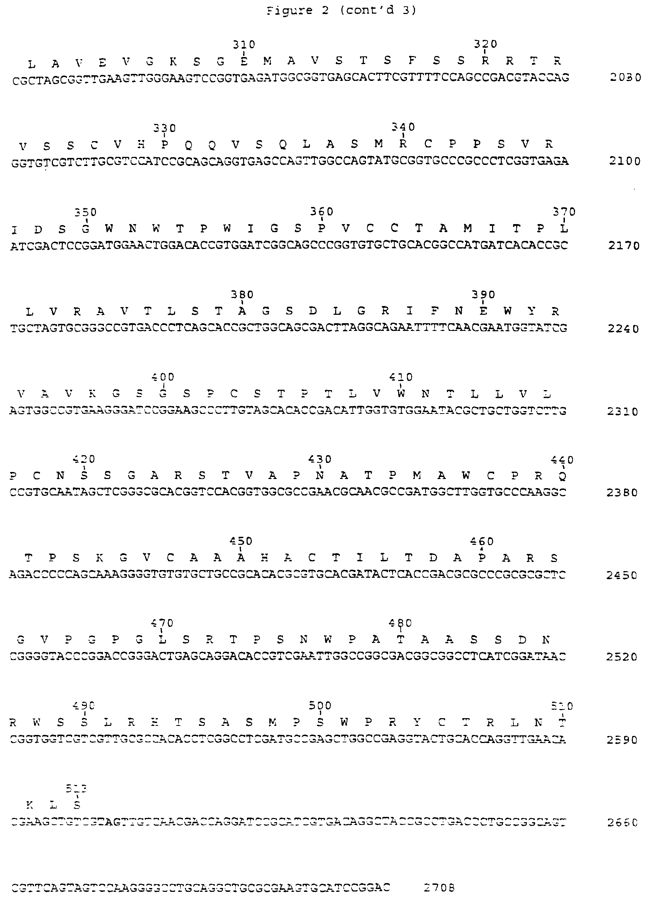

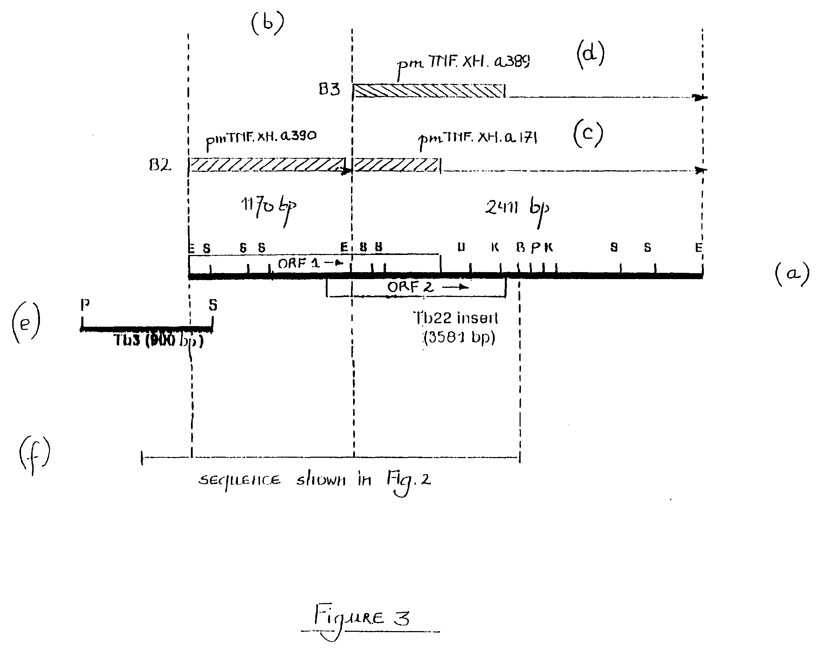

The amino acid sequence of protein B2 (SEQ ID N0 2) is shown in figure 2, and corresponds to the translation product of ORF1 (amino acid sequence shown below the nucleic acid sequence: position 1 to 626).

The amino acid sequence of protein B3 (SEQ ID N0 3) is shown in figure 2, and corresponds to the translation product of ORF2 (amino acid sequence shown above the nucleic acid sequence: position 1 to 513).

It is to be understood that the amino acid sequence of the protein homologous to B2 (or B3) differs from the sequence represented by SEQ ID N0 2 (respectively SEQ ID N0 3) by at least one conservative amino acid substitution as shown in Table 1. The percentage substitutions calculated over the total length of the polypeptide or peptide considered should not exceed 50%, and preferably is lower than 40%, most preferably lower than 30%, 20% or even lower than 10%. The homologous proteins of the invention are all recognized by sera from tuberculous patients.

The fragments of the above-described nucleic acid molecule should encode at least one epitope of the B2 and/or B3 protein. Preferably, the nucleic acid fragment encodes a *M. tuberculosis* (*M. bovis*) specific epitope of the B2 and/or B3 protein. The nucleic acid fragments of the invention contain a stretch of at least 15, preferably at least 30, more preferably at least 45 contiguous nucleotides of the sequence of the nucleic acid molecule as above-defined.

The B2 and B3 antigens described in the current invention form part of the thermostable macromolecular antigen complex (TMA) of *M. tuberculosis (M. bovis),* also termed A60. Purification of the A60 antigen of *M. bovis* BCG has been described before (Cocito et al., 1986; Cocito et al., 1991). The protein components of the TMA complex are responsable for its immunodominant character (Cocito et al., 1995; Fabre et al., 1986). The A60 antigen complex of *M.bovis* has been used as a reagent for an ELISA-type assay (Baelden et al., 1990) and cutaneous testing (Benoit et al., 1989) for the detection of tuberculosis, These assays proved to be able to correctly diagnose infection and to monitor development of disease (Carlucci et al,. 1993; Zou et al., 1994a, 1994b). However, they lacked species-specificity, owing to the extensive cross-reactions among homologous proteins of different mycobacterial species (Coetsier et al., 1994).

The aim of the current invention was to identify those protein components of the A60 complex which contain immunodominant epitopes specific for the species *M. tuberculosis* (and *M. bovis*), to clone and sequence the corresponding gene(s), and to produce the proteins (or protein fragments) by recombinant DNA techniques. As will be shown in the examples section, this approach has enabled the identification of a *M. tuberculosis* genomic fragment containing two overlapping genes (b2 and b3), whose translation products are both recognized by tuberculous patients sera. The DNA segment including the two genes and their regulatory sequences has been cloned and sequenced, leading to the identification of two open reading frames (ORF 1 and 2), accounting for the synthesis of two proteins B2 and B3. The expression of the two genes from genetically engineered constructs has been demonstrated. Finally, the part of the B2 protein containing species-specific epitopes has been produced in *E. coli* by recombinant DNA techniques, it has subsequently been purified and used as a reagent in a serological assay (B2-ELISA) for the detection of tuberculosis.

According to a more specific embodiment, the invention provides for an isolated nucleic acid as defined above, wherein said nucleotide sequence is selected from:
(i) the nucleotide sequence as represented in figure 2 (SEQ ID N0 1),
(ii) a nucleotide sequence which hybridizes to the nucleotide sequence of (i) and which encodes a B2 protein, or a protein homologous to B2, and/or a B3 protein, or a protein homologous to B3, with said B2 (B3) protein and its homologues as defined above,
(iii) a nucleotide sequence which is degenerate as a result of the genetic code to any of the nucleotide sequences as defined in (i) and (ii) and which encodes a B2 protein, or a protein homologous to B2, and/or a B3 protein, or a protein homologous to B3, with said B2 (B3) protein and its homologues as defined above, and
(iv) a fragment of any of the nucleotide sequences as defined in (i) to (iii), said fragment containing a stretch of at least 15, preferably 30 and more preferably 45 contiguous nucleotides of any of the nucleotide sequences as defined in (i) to (iii), and encoding at least one epitope of the B2 and/or B3 protein, or the homologous proteins thereof.
It should be clear that the term "hybridizing" in point (ii) of the abovementioned embodiment refers to hybridization under stringent conditions, i.e. conditions allowing selective hybridization of nucleic acids which show at least 70% homology, preferably 80% homology, and most preferably 90%, or 95% homology.

The terms "homologous" and "homology" are used in the current invention as synonyms for "identical" and "identity"; this means that amino acid sequences which are e.g. said to be 55% homologous, show 55% identical amino acids in the same position upon alignment of the sequences. The same definition holds for homologous nucleic acid sequences, i.e. nucleic acid sequences which are e.g. said to be 55% homologous, show 55% identical basepairs in the same position upon aligment of the sequences.

According to a more specific embodiment, the invention provides for a nucleic acid as described above, containing or consisting of the nucleic acid sequence as represented in figure 2 (SEQ ID N0 1), or a fragment thereof said fragment containing a stretch of at least 15, preferably at least 30 and more preferably at least 45 contiguous nucleotides of the sequence represented by SEQ ID N0 1, and encoding at least one epitope of the B2 and/or B3 antigen.

More specifically, the invention provides for an isolated nucleic acid molecule as described above, containing a nucleotide sequence extending from position (x) to position (y) in the sequence of figure 2 (SEQ ID N0 1), with (x) and (y) representing any of the following values:
- from position (71) to position (1951),
- from position (398) to position (1951),
- from position (1063) to position (2604),
- from position (272) to position (1438),
- from position (1439) to position (1951),
- from position (1438) to position (2604),
   or a fragment of said nucleic acid, said fragment containing at least 15, preferably at least 30 and more preferably at least 45 contiguous nucleotides of the sequence of said nucleic acid, and encoding at least one epitope of the B2 and/or B3 antigen.

The above-defined nucleotide sequence stretches (x)-(y) encode the following polypeptides or polypeptide fragments:
- the nucleic acid fragment (71 )-(1951) encodes the B2 protein in its longest form, i.e. starting from the first presumable start codon,
- the nucleic acid fragment (398)-(1951) encodes the B2 protein in its shorter form, i.e. starting from the second presumable start codon,
- the nucleic acid fragment (1063)-(2604) encodes the B3 protein,
- the nucleic acid fragment (272)-(1438) encodes the a390 fragment of the B2 protein, described further in the examples section,
- the nucleic acid fragment (1439)-(1951) encodes the a171 fragment of the B2 protein, described further in the examples section,
- the nucleic acid fragment (1438)-(2604) encodes the a389 fragment of the B3 protein, described further in the examples section.

According to another embodiment, the invention provides for a recombinant vector, for cloning and/or expression purposes, into which a nucleic acid as described above, or a fragment of said nucleic acid, has been inserted and wherein, in case of an expression vector, the coding sequence of said nucleic acid is operably linked to an expression cassette enabling the expression of the coding sequence by a host cell.

The expression "operably linked" refers to a juxtaposition wherein the components are configured so as to perform their usual function. Thus, control sequences operably linked to a coding sequence are capable of effecting the expression of the coding gene.

The term "expression cassette" encompasses a cassette of elements enabling the correct transcription and translation of the encoded sequences by the host cell machinery: i.e. a promoter sequence, a ribosome binding site, and possibly also regulating sequences, or secreting signals.

The invention furthermore provides for a host cell transformed by a recombinant vector as described above, with said host cell being preferably a prokaryotic organism, preferably chosen from among the following group of organisms: *E. coli, Streptomyces* species, *Corynebacterium* species, such as e.g. *C. glutamicum*, or fast-growing *Mycobacterium* species, such as e.g. *M. smegmatis*.

Suitable vectors and host cell systems for expression and/or cloning of the nucleic acids of the invention are well known in the art of recombinant DNA technology (e.g. Sambrook et al., 1989; Bothwell et al., 1990), and will be exemplified furtheron.

According to a subsequent embodiment, the invention provides for a polypeptide encoded by any of the nucleic acids as described above, or a fragment thereof, said fragment being recognized by a pool of sera from tuberculous patients.

More particularly, the invention provides for a polypeptide as described above, having at least one of the following amino acid sequences:
(I) the amino acid sequence of protein B2 represented by SEQ ID N0 2,
(ii) the amino acid sequence of protein B3 represented by SEQ ID N0 3,
(iii) the amino acid sequence extending from position (110) to position (626) in SEQ ID N0 2,
(iv) the amino acid sequence extending from position (68) to position (456) in SEQ ID N0 2,
(v) the amino acid sequence extending from position (457) to position (626) in SEQ ID N0 2,
(vi) the amino acid sequence extending from position (126) to position (513) in SEQ ID N0 3,
   or a fragment of any of the polypeptides according to (i) to (vi), said fragment consisting of a contiguous stretch of at least 6, preferably 7, 8, 9, 10, 11, 12, 13, 14, 15, 16, 17, 18, 19, 20, 21, 22, 23, 24, 25, 30, 50 or more amino acids of the sequence represented by SEQ ID N0 2 or SEQ ID N0 3, and being recognized by a pool of sera from tuberculous patients.

According to a very specific embodiment, the invention provides for a peptide having at least one of the following amino acid sequences:
KGADVDAGGSQHNR (K-14-R peptide, SEQ ID N0 4), or
GQPPSRRRGDRHQSTRR (G-17-R peptide, SEQ ID N0 5).

The peptides K-14-R and G-17-R correspond to an epitope region in the B2 (resp. B3) protein. The positions of both peptides are indicated on the sequence of figure 2 (boxed) and on the hydrophilicity patterns of figure 8 (resp. Figure 9).

According to another embodiment, the invention provides for a fusion protein comprising an amino acid sequence of a polypeptide as described above, or a fragment thereof, linked to a heterologous amino acid sequence.

The production of the recombinant polypeptides of the invention as fusion proteins may offer several advantages, such as e.g. enhanced expression levels of the polypeptides of the invention, or easiness of purification due to the presence of purification "tags" , or a better presentation of the polypeptide on a solid surface. The heterologous part of the fusion protein may be cleaved off in a later stage using appropate endoproteases, or it may remain attached to the polypeptides of the invention.

In order to carry out the expression of the polypeptides of the invention in bacteria, such as *E. coli*, the following steps are required:
- transformation of an appropriate bacterial host strain with a recombinant vector in which a nucleotide sequence coding for one of the polypeptides of the invention has been inserted (insert) under the control of the appropriate regulatory elements, particularly a promoter recognized by the polymerases of the bacterial host and an appropriate ribosome binding site (RBS), enabling the expression in said bacterial host of said nucleotide sequence,
- culture of said transformed bacterial host in conditions enabling the expression of said insert, and
- possibly, purification of the recombinant polypeptide.

The techniques for carrying out the expression of recombinant proteins in any of the other hosts as specified above are also well known in the art of recombinant expression technology.

The invention thus also provides for a method for the production of a recombinant polypeptide as described above, or a fusion protein as described above, comprising at least the following steps:
- culturing a transformed cellular host as described above under conditions allowing the heterologous expression of the polypeptide which is encoded,
- isolating the polypeptide from the culture broth.

According to another embodiment, the invention provides for an antibody raised against a polypeptide as described above, more preferably a monoclonal antibody. Antibodies according to the invention include specific polyclonal antisera prepared against the *M. tuberculosis* polypeptides of the invention, and having no cross-reactivity to other proteins. The antibodies according to the invention specifically include monoclonal antibodies raised against the *M. tuberculosis* polypeptides of the invention.

The monoclonal antibodies of the invention can be produced by any hybridoma liable to be formed according to classical methods from splenic cells of an animal, particularly of a mouse or rat, immunized against the polypeptides of the invention on the one hand, and of cells of a myeloma cell line on the other hand, and to be selected by the ability of the hybridoma to produce the monoclonal antibodies recognizing the polypeptides which have been initially used for the immunization of the animals.

The monoclonal antibodies according to this preferred embodiment of the invention may be humanized versions of the mouse monoclonal antibodies made by means of recombinant DNA technology, departing from the mouse and/or human genomic DNA sequences coding for H and L chains or from cDNA clones coding for H and L chains.

Also fragments derived from these monoclonal antibodies such as Fab, F(ab)'₂ and ssFv ("single chain variable fragment"), providing they have retained the original binding properties, form part of the present invention. Such fragments are commonly generated by, for instance, enzymatic digestion of the antibodies with papain, pepsin, or other proteases. It is well known to the person skilled in the art that monoclonal antibodies, or fragments thereof, can be modified for various uses.

The antibodies of the current invention can be labelled by an appropriate label of the enzymatic, fluorescent, or radioactive type.

According to another embodiment, the invention provides for a method for the *in vitro* detection of tuberculosis disease in a mammal subject, based on the detection of anti- *M. tuberculosis* antibodies present in a sample taken from said subject, comprising the steps of
- contacting said sample with a polypeptide as described above, or with a fusion protein as described above, under conditions allowing the formation of an immunological complex, and
- detecting the presence of an immunological complex formed between said polypeptide and the anti - *M. tuberculosis* antibodies which may be present in said sample.

It is to be understood that the detection of anti- *M. tuberculosis* antibodies in a serum sample is an indication of tuberculosis disease.

Preferably said mammal is a human subject, or a bovine animal.

The sample may be any body fluid possibly containing antibodies against *M. tuberculosis*, such as cerebrospinal fluid, serum, blood, plasma, milk, urine... Preferably, the sample is serum.

Conditions allowing the formation of an immunological complex are known to the person skilled in the art.

According to an alternative embodiment, the invention provides for a method for the *in vitro* detection of tuberculosis disease in a mammal subject, based on the detection of *M. tuberculosis* antigens present in a sample taken from said subject, and comprising the steps of
- if necessary, pretreatment of said sample in order to release the *M. tuberculosis* antigens which may be present in said sample,
- contacting said sample with an antibody as described above, under conditions allowing the formation of an immunological complex, and
- detecting the presence of an immunological complex formed between said antibody and the *M. tuberculosis* antigens which may be present in said sample.

It is to be understood that the detection of *M. tuberculosis* antigens in a sample is an indication of tuberculosis disease.

Design of immunoassays suitable for detection of antibodies or antigens in a sample, is subject to a great deal of variation, and many formats are known in the art. Most assays involve the use of labeled antibody and/or polypeptide. The labels may be, for example, enzymatic (like horse radish peroxidase, alkaline phosphatase), fluorescent, chemoluminescent, radioactive, or dye molecules. In an enzyme linked immuno sorbent assay (ELISA) the label is enzymatic, and the detection signal is usually colorimetric (optical density).

Different types of ELISA may be feasable for detection of antibodies in a sample. In an indirect ELISA the polypeptides of the invention are coated on a solid surface (microtiter plates) where they capture the antibodies possibly present in the sample. The bound antibodies from the sample are subsequently detected by a labeled second antibody (conjugate). The signal generated by the latter is quantified.

In an antigen sandwich ELISA, the polypeptides of the invention are used twice, first as a capturing agent (bound on the surface of e.g. a microtiter plate) and secondly as a detecting agent (labelled polypeptide reacting with the bound antibodies from the sample). The antibodies from the sample are "sandwiched" between two layers of peptide (this is made possible by the two antigen binding sites which are present on each antibody).

In an antibody sandwich ELISA, the antibodies of the invention are used twice, first as a capturing agent (bound on the surface of a solid phase) and secondly as a detecting agent (labelled antibody reacting with the bound antigen from the sample). The antigens to be detected from the sample are "sandwiched" between two layers of antibodies.

According to a preferred embodiment, the invention provides for an immunodetection method or assay as described above, wherein said method or assay is an ELISA.

According to another embodiment, the invention provides for an immunodiagnostic kit for the in vitro detection of tuberculosis disease in mammals, said kit comprising a polypeptide as described above, or a fusion protein as described above, or an antibody as described above.

Acccording to a more specific embodiment, the invention provides for an immunodiagnostic kit for the *in vitro* detection of tuberculosis disease in mammals, based on the detection of anti- *M. tuberculosis* antibodies present in a sample taken from said mammal, said kit comprising a polypeptide as described above, or a fusion protein as described above, possibly in combination with other *M. tuberculosis* antigens.

In a preferred embodiment, the immunodiagnostic kit comprises a solid support onto which the polypeptide of the invention has been readily immobilized.

The solid support onto which the polypeptides of the invention are coated may be chosen from the supports commonly used in the art of immunodiagnosis. Depending on the type of assay the solid support may be a polystyrene carrier (microtiter plate (e.g. ELISA) or beads (e.g. microbeads assay)), a nylon membrane (LIA (Line Immuno Assay)), latex beads (coagulation tests), etc. If different polypeptides are used simultaneously as detecting reagent, they may be coated either on different locations of the solid support, or together, on the same location.

The present invention relates more particularly to a kit for determining the presence of anti- *M. tuberculosis* antibodies in a biological sample liable to contain them, comprising:
- at least one polypeptide as defined above, preferentially in combination with other polypeptides or peptides from *M. tuberculosis*, with said polypeptides being preferentially immobilized on a solid substrate,
- a buffer or components necessary for producing the buffer enabling a binding reaction between these polypeptides and the antibodies against *M. tuberculosis* present in the biological sample,
- means or reagents for detecting (i.e. generating a detectable signal from) the immune complexes formed in the preceding binding reaction,
- possibly also including an automated scanning and interpretation device for inferring the presence of *M. tuberculosis* antibodies in the sample from the detected signal.

According to another embodiment, the invention provides for a diagnostic kit for the detection of *M. tuberculosis* antigens in a sample, said kit comprising an antibody as described above.

According to yet another embodiment, the present invention relates to a method using at least one of the polypeptides of the invention, possibly in combination with other *M. tuberculosis* polypeptides, for measuring a cellular immune response in an individual which has been in contact with the *M. tuberculosis* pathogen, said cellular immune response being measured either *in vivo*, such as a delayed type hypersensitivity reaction upon subcutaneous injection of the polypeptides of the invention, or *in vitro*, such as the stimulation of periferal blood lymphocytes or the secretion of certain lymphokines or cytokines (e.g. interferon-gamma), upon addition of the polypeptides of the invention to a sample of periferal blood lymphocytes under conditions allowing recognition of the polypeptides by the cells responsive for the immune response, conditions which are known to the person skilled in the art.

It is to be understood that the presence of a cellular immune response against *M. tuberculosis* antigens in a subject, indicates that the subject has been in contact with the *M. tuberculosis* pathogen. It is an indication of a protective immune response which has been mounted against the pathogen, and it is not necessarily an indication of tuberculosis disease. This is in contrast with the presence of anti-*M.tuberculosis* antibodies, or the presence of *M. tuberculosis* antigens in a sample, which do indicate the presence of tuberculosis disease in the subject from which the sample was taken, as described above.

The invention also provides for a vaccine composition for the prevention of tuberculosis disease in mammals, said vaccine composition comprising as an active principle a polypeptide as described above, or a fusion protein as described above, or a nucleic acid as described above, or a recombinant vector as described above, said active principle being combined with a pharmaceutically acceptable carrier.

Besides the *M. tuberculosis* polypeptides according to the invention, said vaccine composition may comprise also any other *M. tuberculosis* immunogenic component or any other bacterial or other immunogenic component in general.

In a specific embodiment, the nucleic acid encoding any of the polypeptides as defined above, is used as a vaccine, either as naked DNA or as part of recombinant vectors. In this case, it is the aim that said nucleic acid is expressed into immunogenic protein / peptide *in situ* in the vaccinated host and thus confers *in vivo* protection (e.g. Ulmer et al., 1993).

The active ingredients of such a vaccine composition may be administered orally, subcutaneously, conjunctivally, intramuscularly, intranasally, or via any other route known in the art including for instance via the binding to carriers, via incorporation into liposomes, by adding adjuvants known in the art, etc.

### FIGURE LEGEND

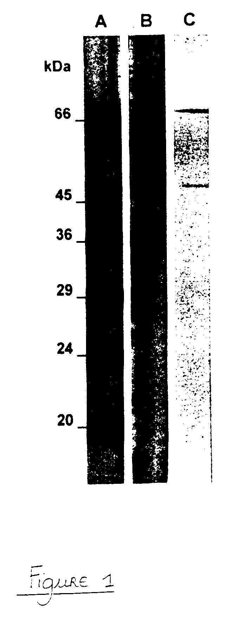

**Figure 1**. Immunoblot analysis of recombinant polypeptides produced by the ZAP-Tb22 phage. Proteins obtained, upon IPTG-induction, from the lysis plaques were separated by acrylamide gel electrophoresis (12% SDS-PAGE), and transferred to nitrocellulose membranes, which were either stained with colloïdal gold (lane B), or incubated with a pool of tuberculous patients sera (lane C). Molecular weight markers are in lane A.
**Figure 2.** Nucleotide sequence (SEQ ID N0 1) of the overlapping genes b2 and b3 encoding the B2 and B3 proteins of *M. tuberculosis*. The nucleotide sequence shown in this figure contains two open reading frames of 1878 (ORF1) and 1539 (ORF2) nucleotides. The putative initiation codons and ribosomal binding sites are indicated in bold characters. Promoter-like sequences are underlined. The b2 and b3 genes encode proteins of 626 and 513 aa respectively, whose deduced amino acid sequences are displayed. The lower amino acid backbone represents the protein B2 (SEQ ID N0 2). The upper amino acid backbone represents the protein B3 (SEQ ID N0 3). H* indicates the first amino acid residue encoded by the *M.tuberculosis* DNA insert within the ZAP-Tb22 phage. The EcoR1 and SaI1 restriction sites used for subcloning are underlined. The protein fragments a390, a171 and a389 which have been expressed in *E. coli* are indicated with arrows. The peptides K-14-R (B2) and G-17-R (B3) are boxed. The position of the nucleic acid region hybridizing to the Tbbiot1 probe is also indicated (double underlined).

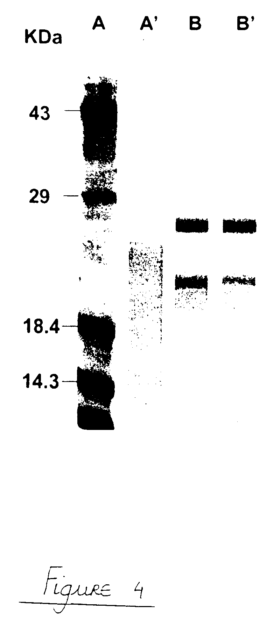

**Figure 3**. Schematic diagram of subclones used for expression and/or sequencing of portions of the b2 and b3 genes.
   (a) Scheme of the Tb22 DNA insert indicating the positions of the ORF 1 and 2, and of the main restriction sites EcoR1=E, Sal1=S, Kpn1=K, BamH1=B, Pst1=P) used for subcloning.
   (b) The 1170-bp EcoR1 fragment of Tb22 was ligated into the EcoR1 site of pmTNFIIXH, yielding the pmTNF.XH.a390 plasmid carrying the first 390 codons of ORF1, under the control of the pl lambda promoter. The insert of the plasmid is shown in shaded box.
   (c) (d) The adjacent 2411 -bp EcoR1 fragment was ligated into the EcoR1 site of pmTNFIIXH and pmTNFIIIXH. The resulting plasmids, carried the terminal 171 codons of ORF1 (pmTNF.XH.a171), and the 389 carboxyl-terminal codons of ORF2 (pmTNF.XH.a389), respectively, under the control of the pl lambda promoter. The inserts of the plasmids are shown in shaded boxes.
   (e) position of the Tb3 clone (Pst1-Sal1) carrying the upstream region and presumptive starting codons of the b2 gene.
   (f) position of the region of which the nucleic acid sequence is illustrated in figure 2.

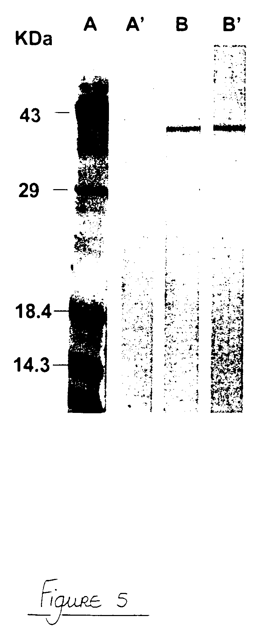

**Figure 4**. Western blot analysis of the recombinant polypeptide a171. Crude cell extracts from *E.coli* cells containing the pmTNF.XH.a171 plasmid were heat-shocked at 42°C, fractionated by 12% SDS-PAGE, transferred to nitrocellulose membranes, and reacted with a pool of tuberculosis patient sera, preabsorbed (lane B 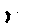 ) or not (lane B) with a *M.avium* lysate. Lane A 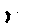 : lysate of *E.coli* DH5α carrying no mycobacterial insert (negative control). Lane A : molecular mass standards.

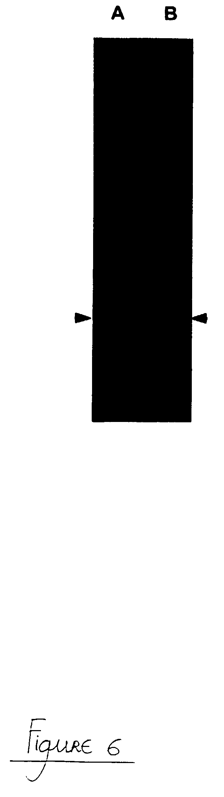

**Figure 5**. Western blot analysis of the recombinant polypeptide a389. Crude cell extracts from *E.coli* cells containing the pmTNF.XH.a389 plasmid were heat-shocked at 42°C, fractionated by 12% SDS-PAGE, transferred to nitrocellulose membranes, and reacted with the pool of tuberculosis patients sera, preabsorbed (lane B 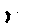 ) or not (lane B) with *M.avium* lysates. Lane A 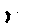 : lysate of *E.coli* DH5αcarrying no mycobacterial insert (negative control). Lane A : molecular mass standards.

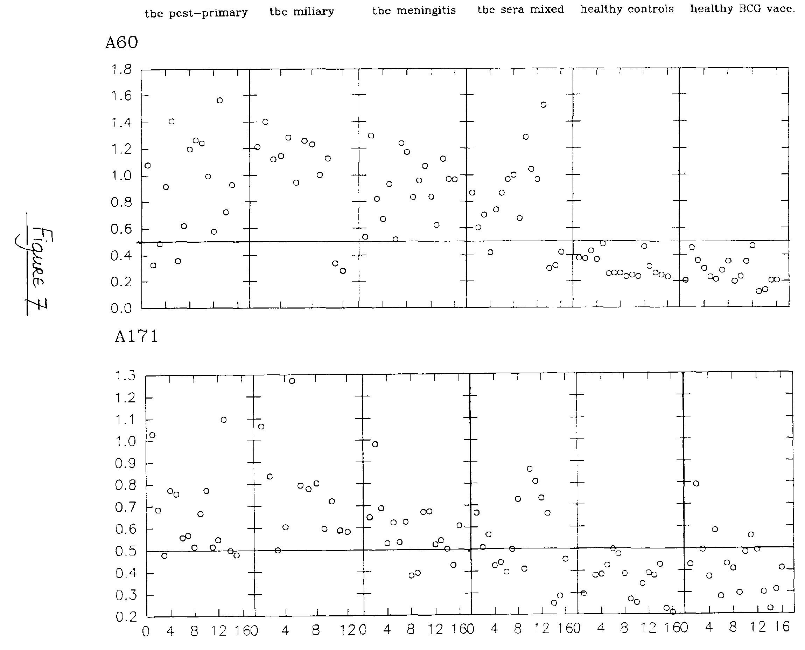

**Figure 6.** Electrophoretic analysis of the proteins expressed by recombinant E. coli, containing the plasmid pmTNF.XH.a171. The inclusion bodies fraction from recombinant *E.coli* SG4044 cells (containing the pmTNF.XH.a171 plasmid that codes for the carboxyl-terminal portion of protein B2) were isolated. Dissociated proteins were fractionated by electrophoresis (12% SDS-PAGE), and stained with Coomassie blue. (A) total cell lysate; (B) inclusion bodies.
**Figure 7**. Comparative serological analysis of healthy (BCG vaccinated and non-vaccinated) and tuberculosis patients sera by the A60 and the a171 ELISA assays. The absorbance values (A₄₉₂nm) are reported on the ordonate axis. The horizontal lines represent the cutoff values. Different types of tuberculous (tbc) patients sera were considered: tbc post-primary (= patients who relapse after a previous tb-infection), tbc miliary (=patients with disseminated form of the disease throughout the body), tbc meningitis (=patients from which tuberculous bacilli have been isolated from the cerebrospinal fluid), tbc sera mixed (=patients with tuberculous infection of different organs, besides a pulmonary tuberculosis).

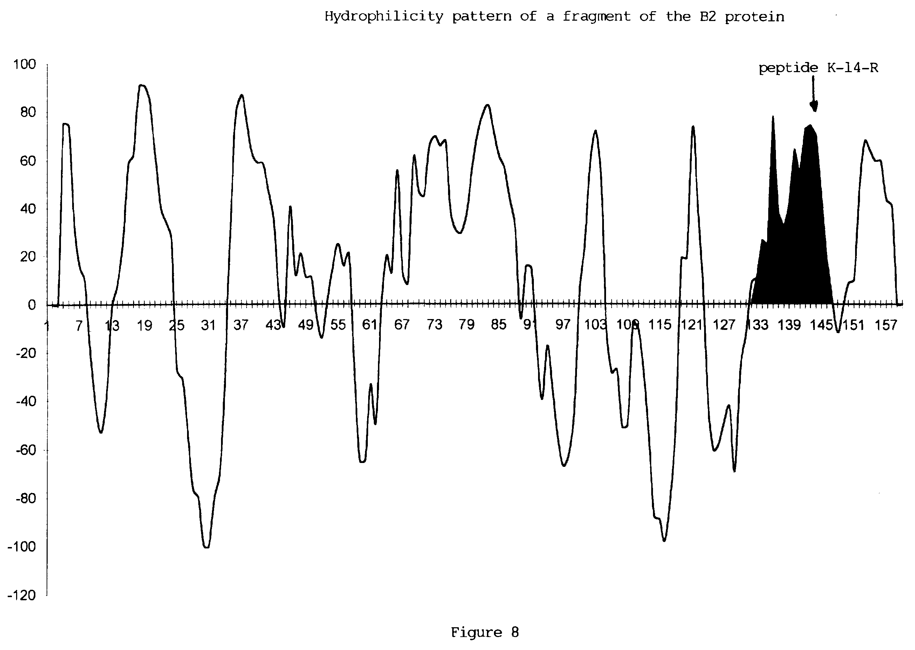

**Figure 8:** Hydrophilicity pattern of the B2 protein. Hydrophilicity values on the ordinate axis are calculated by the combined methods of Parker et al. 1986 (hydrophilicity *sensu strictu*), Karplus et al. 1985 (flexibility) and Janin, 1979 (accessibility). The region of the peptide K-14-R is indicated in black. Position 1 in this figure corresponds to amino acid position 455 in the B2 protein sequence as shown in figure 2.

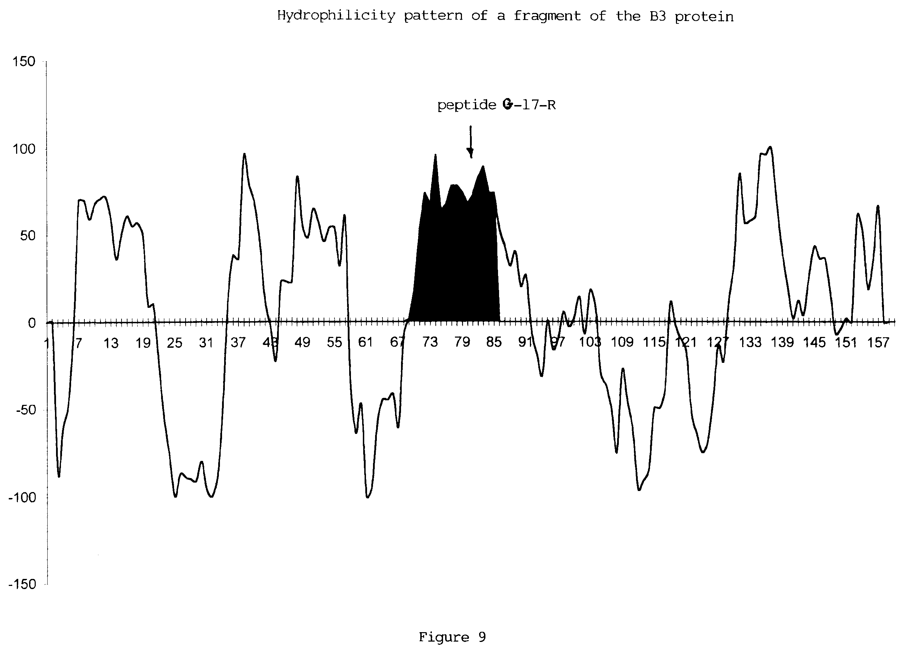

**Figure 9:** Hydrophilicity pattern of the B3 protein. Hydrophilicity values on the ordinate axis are calculated by the combined methods of Parker et al. 1986 (hydrophilicity *sensu strictu*), Karplus and Shultz, 1985 (flexibility) and Janin, 1979 (accessibility). The region of the peptide G-17-R is indicated in black. Position 1 in this figure corresponds to amino acid position 125 in the B3 protein sequence as shown in figure 2.

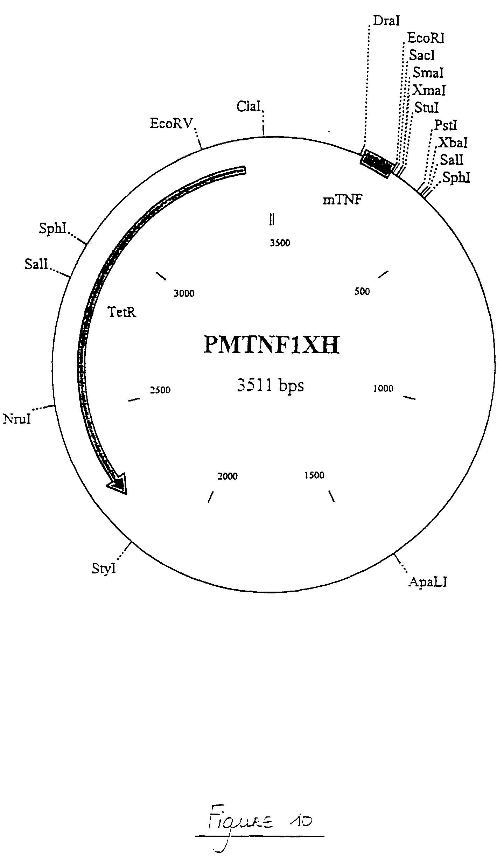

**Figure 10**: Map of the pmTNFIXH plasmid used for expression of the polypeptides of the invention as a N-terminal fusion protein with the first 25 amino acids of mTNF. The plasmids pmTNFIIXH and pmTNFIIIXH are identical to pmTNFIXH except for the sequence of the polylinker site which contains one (pmTNFIIXH) or two (pmTNFIIIXH) additional nucleotides, allowing the translation of the different reading frames (I, II, III) of an inserted nucleic acid molecule. A detail of the nucleotide sequence of the polylinker site for the three different plasmids is given below:

polylinker site of pmTNFIXH:

Sac I Smal
... CAG GGA ATT CGA GCT CGG TAC CCG GGG AGG CCT GCA CGT GAT CGA
aa 25 mTNF EcoRI KpnI StuI PmlI

GGG CAG ACA CCA TCA CCA CCA TCA CTA ATA GTT AAT TAA CTG CAG GGA
PacI PstI

HindIII
TCC TCT AGA GTC GAC CGG CAT GCA AGC TTA AGT AAG TAA ...
BamHI XbaI SalI SphI

(SEQ ID N0 7)

polylinker site of pmTNFIIXH:

Sac I Smal
... CAG CCG AAT TCG AGC TCG GTA CCC GGG GAG GCC TGC ACG TGA TCG
aa 25 mTNF EcoRI KpnI StuI PmlI

PstI
AGG GCA GAC ACC ATC ACC ACC ATC ACT AAT AGT TAA TTA ACT GCA GGG
PacI

XbaI HindIII
ATC CTC TAG AGT CGA CCG GCA TGC AAG CTT AAG TAA GTA A ...
BamHI SalI SphI

(SEQ ID N0 8)

polylinker site of pmTNFIIIXH:

SacI Smal
... CAG CCC GAA TTC GAG CTC GGT ACC CGG GGA GGC CTG CAC GTG
aa 25 mTNF EcoRI KpnI StuI PmlI

PstI
ATC GAG GGC AGA CAC CAT CAC CAC CAT CAC TAA TAG TTA ATT AAC TGC
PacI

XbaI HindIII
AGG GAT CCT CTA GAG TCG ACC GGC ATG CAA GCT TAA GTA AGT AA ...
BamHI SalI SphI

(SEQ ID N0 9)

### EXAMPLES

### I. Materials and methods

**Bacterial strains.** The following mycobacteria were used: *M.tuberculosis* H37Rv, MAIS ATCC-10708, MAIS ATCC-10719, *M.avium* D4 (from F. Portaels, Institute of Tropical Medecine, Antwerp, Belgium) and *M. scrofulaceum* 82/549 (from M. Weckx, Pasteur Institute, Brussels, Belgium). *A M.leprae* preparation was provided by the World Health Organization (WHO, Geneva, Switzerland).

The strains of *Escherichia coli* K-12 DH5αF 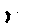 (Life Technologies, Gaithersburg, Md.), XL1-Blue MRA (Stratagene, La Jolla, Calif.) and SG4044 (Innogenetics, Ghent, Belgium) were used as hosts for cloning and/or expression.

**Antibodies and sera.** Sera from tuberculosis patients were obtained from the Pneumology Department, Mt. Godinne Clinics, Belgium. Five sera from human immunodeficiency virus-negative postprimary tuberculosis cases under chemotherapy were pooled and used for serological screening.

Polyclonal rabbit antibodies against the peptides K-14-R (from B2 protein) and G-17-R (from B3 protein) were raised according to the techniques well known in the art. The peptides were coupled with KLH carrier protein (Neosystem Labs, Strasbourg, France). Conjugate KLH-peptides (500 µg) emulsified with incomplete Freunds adjuvant were subcutaneously inoculated to rabbits twice, at two weeks interval. Sera were taken two weeks after the second injection. They were tested by Western blot analysis on BCG lysates (results not shown).

**Serum absorption.** At all stages of the work, sera were previously absorbed on lysates of *E.coli* K-12 (the cloning host). To identify species specific epitopes, cross-reacting antibodies were eliminated by absorption on lysates of atypical mycobacteria. Sera diluted (1/50) in TBS buffer (0.15 M NaCl, 0.10 M Tris-HCL [pH 7.5] containing 0.05 % [vol/vol] Tween 20, 10 mM phenylmethylsulfonyl fluoride, and 1 % [wt/vol] gelatin) were incubated overnight at 4°C with bacterial lysates (5 mg [dried weight] per ml of diluted serum). Absorption efficiency was controlled by dot-blot for Western blot analysis, and by ELISA for serological testing.

**DNA techniques.** Chromosomal DNA from *M.tuberculosis* H37Rv was prepared as previously described (De Kesel et at. 1993). Purification of phage DNA, DNA digestion, ligation, transformation of *E.coli* cells with plasmid, and infection with phage DNA were carried out according to Sambrook et al. (1989). The boiling minilysate method (Holmes et al. 1981) was used to screen restriction endonuclease segments of plasmid DNA. Large-scale preparation of *E.coli* plasmid DNA was made using a Qiagen-tip 500 kit (Westburg b.v., Leusden, Netherlands), Restriction enzymes and T4 DNA ligase were purchased from Boerhinger (Mannheim, Germany). Restriction endonuclease fragments were purified from agarose gels with Nucleotrap kit (Macherey-Nagel, Düren, Germany).

Screening of bacteriophage plaques and Southern blot analysis (Sambrook et al., 1989), were done with the biotinylated DNA probe Tbbiot1 (5 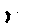 -bGCGGGCGTTTCGGCTTCACC (SEQ ID N0 6)) and nylon membranes (Hybond-N+, Amersham, Buckinghamshire, UK).

**Nucleotide sequence analysis.** For analysis of DNA sequences, appropriate restriction fragments of cloned inserts were subcloned within the polylinker sites of pBluescript SK⁻ (Stratagene, La Jolla, California), Subclone sequences were determined by the dideoxy chain termination method by using the Taq Dye Deoxy Terminator Cycle sequencing kit and a ABI 373a DNA sequencer (PE Applied Biosystems, Foster City, CA). The complete sequence was obtained with the M13 forward and reverse primers.

**Expression of ORFs by** ***E.coli*****.** Recombinant plasmids were introduced in *E.coli* SG 4044 by transformation. Single transformed colonies were grown at 28°C in the presence of tetracycline to an A₆₀₀ of 0.2, and then heat shocked (4h, 42°C). Cell lysates were mixed with 4x SDS gel-loading buffer and analyzed by Western blot.

**Purification of a171 polypeptide.** *E.coli* SG 4044 cells containing the pmTNF-a171 plasmid, were concentrated by centrifugation and lysed by the lysozyme-plus-detergent procedure. The inclusion bodies were pelleted by centrifugation, washed with Triton X-100 and EDTA, and solubilized with 8 M urea (Sambrook et al. 1989).

**SDS-PAGE and Western blot analysis.** Different protein samples (lysis plaques, or cell lysates, or a171 polypeptide) in gel loading buffer, were boiled at 100°C for 5 min and loaded on 12 % polyacrylamide gels. Protein fractionation was carried out at a constant current of 30 mA in a vertical electrophoresis unit (gel 16 by 18 cm, Hoefer Scientific Instruments, San Francisco, Cal.). Electrophoresed components were transblotted (LKB 217 Multiphor 2, Bromma, Sweden, 100 mA, 2 h, with buffer containing 20 % methanol, 0.039 M glycine and 0.048 M Tris base pH 8.8) onto nitrocellulose membranes (BA 85, Macherey-Nagel, Germany). Total proteins were stained by colloïdal gold (Aurodye forte, Amersham, UK). Tuberculosis-specific proteins were visualized by sequential incubation with a pool of tuberculosis patients sera and with peroxydase-labeled anti-human immunoglobulins.

### II. Isolation and characterization of the DNA fragment containing the b2 and b3 genes of M. tuberculosis

**Construction and immunological screening of a** ***M.tuberculosis*** **genomic library.** A genomic library of *M.tuberculosis* H37Rv was constructed as previously described (De Kesel et al. 1993). Mycobacterial DNA fragments of 2 to 5 kb were ligated with lambda ZAPII (Stratagene, La Jolla, Calif.), by using *EcoRl* linkers. Recombinant lambda phages from the unamplified genomic library were plated according to manufacturers directions. After incubation at 42°C, when lysis plaques were just seen, plates were covered with nitrocellulose filters satured with isopropyl-β-D-thiogalactopyranoside (IPTG), and incubated overnight at 37°C. Filters were washed with TBS buffer (0.15 M NaCl, 0.01 M Tris-HCl pH 7.5), incubated with the same buffer containing 2 % gelatin, and then for 12 h with a pool of 5 tuberculosis patients sera diluted (1/20) in TBST buffer (TBS buffer containing 0.05 % Tween 20 and 1 % gelatin). Bound human Ig were revealed with an anti-human-Ig second serum conjugated with horse peroxydase (Dako, Copenhagen, Denmark). Immunoreactive lysis plaques were detected with chloro-1-naphtol (Rio Rad Laboratories, Richmond, Calif.) and H₂O₂ (0.015%). The plaques corresponding to the reactive spots on the filters were transferred to SM medium (100 mM NaCl, 10 mM MgSO₄, 20 mM Tris-HCl pH 7.4), and purified by repeated passages on *E.coli* DH5αF 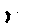 .

Proteins produced by recombinant phages were analyzed by Western blot. Plaque-purified recombinant phages were plated in soft agar, in the presence of 10 mM IPTG. The plates were incubated for 4h at 42°C, and then one night at 37°C. Approximatively 10 plaques of each recombinant phage were removed and suspended in 100 µl of SDS gel-loading buffer (0.125 M Tris-HCl pH 6.8, 5 % SDS, 20 % glycerol, 10% β-mercaptoethanol, and 0.05 % bromophenol blue).

**Isolation of recombinant phages coding for *M.tuberculosis*-specific proteins.** Recombinant lambda ZAPII phages carrying mycobacterial DNA sequences were screened with a pool of tuberculosis patients sera: 21 strongly reactive clones were selected out of some 100,000 plaques. These clones were retested with anti-A60 polyclonal serum: some 16 positive clones were selected, and their expression products were then tested with the same pool of tuberculosis patients sera preabsorbed on lysates of non-tuberculous mycobacteria (MAIS strains, *M. avium*, and *M.leprae* strains). One phage (ZAP-Tb22) expressed a product strongly reacting with patients' sera and specific for *M.tuberculosis*, with respect to the other mycobacterial species. Western blot analysis of proteins from the lysis plaques of the recombinant phage showed two bands of 65 and 55 kDa reacting with tuberculosis patients sera (Fig. 1).

**Analysis of the Tb22 region of recombinant mycobacterial DNA.** Recombinant phage ZAP-Tb22 carrying a mycobacterial DNA segment was amplified. Its DNA was purified and hydrolyzed with EcoR1 endonuclease, yielding a DNA insert of about 3,700 bp (Tb22) as determined by gel electrophoresis. The DNA sequence of the Tb22 insert was determined (see figures 2 and 3).

The expression of Tb22 was analysed by subcloning the restriction fragments of the insert in the members of an expression vectors family (pmTNFXH I, II and III; Innogenetics, Ghent, Belgium). A map of these vectors is shown in figure 10. These constructs allow the translation of the cloned genes in the 3 reading frames, as N-terminal fusion proteins with the first 25 amino acids of mouse tumor necrosis factor α, under the control of the pl lambda promoter (see also Gilot et al. 1993). Three of the analyzed constructs proved to be particularly interesting. Thus, a 1170 bp EcoR1 DNA fragment, subcloned in vector pmTNFIIXH, yielded a recombinant plasmid (pmTNF.XH.a390) which expressed the initial part of a gene comprised in Tb22, from now on called the b2 gene. The adjacent 2411 bp-EcoR1 segment sub-cloned in the same vector (plasmid pmTNF.XH.a171) expressed a further portion (513 bp) of the same gene. However, this same DNA fragment, upon insertion in vector pmTNFIIIXH (plasmid pmTNF.XH.a389), which allows expression of a different reading frame on the same strand, expressed a 1167 bp product, forming part of a second gene comprised in the Tb22 clone (gene b3).

These results indicated that the Tb22 insert of mycobacterial DNA carried two partly overlapping ORF's (ORF1 corresponding to the b2 gene and ORF2 corresponding to the b3 gene), which were transcribed and translated into protein fragments in the recombinant strains. The overall expression product of Tb22 contained two different proteins corresponding to the partly overlapping reading frames ORF1 and ORF2 depicted in figures 2 and 3. The subsequent examples will demonstrate that fragments of both proteins (B2 and B3) are recognized by a pool of tuberculous sera, and contain species specific epitopes useful in the serodiagnosis of tuberculosis.

### III. Characterization and expression of the b2 gene (ORF1)

**Base composition and expression of the B2 gene.** According to the nucleotide sequence of the 3,700 bp Tb22 insert, and to the open reading frame ORF1 (in phase with the α-peptide gene of lambda ZAP), a DNA segment of 1551 bp was delineated (Fig.2). This segment extended from an ATG triplet at position 398 to a TAG triplet at position 1949, representing the putative (second) start and stop signals of gene b2, respectively.

The overal base composition of the b2 gene (63,5 mol% of G+C), was consistent with the global G+C content observed in the mycobacterial genome. The G+C contents for codon positions 1, 2, and 3 were 64.8, 47.5, and 74.3 mol%, respectively. These data agree with the codon usage described for the few known genes of the *M.tuberculosis* complex, and confirm the preference for either G or C in the third position.

A search for regulatory sequences within the nucleotide stretch preceding the b2 start codon at 398, revealed the presence of an AG-rich segment (G₃₈₉AAGG₃₉₃) resembling the Shine-Dalgarno consensus elements found in several bacteria, and also of promoter-like sequences (Hawley and McClure, 1983) mimicking those previously described in *E.coli*.

The α-peptide Tb22 fusion protein (corresponding to the Tb22 insert) had a calculated size of 62,990 Da, in agreement with a molecular mass of about 66 kDa determined by Western blotting (size of α-peptide= 3864 Da). However, the expression of the entire ORF1 in *E.coli* under the control of a strong promoter was unsuccesful, presumably because entailing the synthesis of a protein detrimental for the host cell. To circumvent this difficulty, two plasmids were constructed: pmTNF.XH.a390 containing the first 390 codons of ORF1, and pmTNF.XH.a171 including the 171 carboxyl-terminal codons of ORF1, both expressed under the control of the pl lambda promoter (Fig. 4). Exponential SG4044 cells harboring either plasmid produced relatively high levels of the corresponding gene products, detected as SDS-PAGE bands of 45- and 25-kDa respectively (data not shown), in agreement with the values calculated from the DNA sequence. In Western blot analysis, only the 25-kDa product mTNF-a171 was recognized by the pool of tuberculosis sera (Fig. 4, line C), suggesting the occurrence of B cell epitopes in the carboxyl-terminal portion of the B2 protein. The species-specificity of these epitopes was verified in Western blots with tuberculosis patients sera pre-absorbed with sonicates of either *M.leprae* or *M. avium* strains (Fig. 4, line D). Persistence of the positive signal upon serum pre-absorption proved the B2 protein to have *M.tuberculosis*-specific epitopes non crossreacting with other mycobacterial species.

The hydrophilicity pattern of the B2 protein is illustrated on figure 8. According to this profile a predicted antigenic peptide K-14-R was synthesised. Antibodies were raised against this peptide in rabbits according to the method described in example 1.

**Cloning of the DNA segment preceding the b2 gene**. To confirm the structure of the mycobacterial DNA segment including the origin of the b2 gene, and to explore the possible occurrence of additional regulatory elements in this region, 10- to 12-kb-DNA fragments from a *M.tuberculosis* H37RV gene bank, were ligated with the lambda DASH cloning vector, using BamH1 linkers. One phage, out of some 4,000 plaques screened by *in situ* hybridization with the Tbbiot1 probe (carrying the initial sequence of the Tb22 insert), yielded a 0.9-kb Sal1-Pst1 DNA fragment (Tb3). The latter was subcloned in the vector pbluescript SK- and sequenced. Inspection of the sequence reported in Fig.2 revealed another putative starting codon for the b2 gene at position 71 preceded by a putative Shine-Dalgarno element (G₆₀AGA₆₃).

### IV. Characterization and expresssion of the b3 gene (ORF2)

**Base composition and expression of the b3 gene.** A segment of the Tb22 insert, which would account for a translation product of an open reading frame (ORF2) different than that of b2 was sought in the sequence map of Fig. 2. A 1539 bp-long sequence, partly overlapping that of b2/ORF1 and involving the same strand in the same direction was thus indentified. The corresponding gene (b3) extended from a putative initiation TTG triplet at position 1063 to a presumptive stop codon TAG at position 2602. The initiation codon of b3 was located 665 bp downstream that of b2, with a 886 bp overlap of the two genes. A search for regulatory sequences preceding the TTG₇₉₂ codon suggested the presence of a putative Shine-Dalgarno-like element (G₁₀₅₄GTGG₁₀₅₈), the presumptive translation initiation signal of ORF2.

A plasmid containing the terminal 1167 bp of ORF2 under the control of the pl lambda promoter (pmTNF.XH.a389) was constructed and introduced by transformation in *E. coli* SG4044 cells. The lysates of these recombinant cells, submitted to Western blot analysis with a tuberculous sera pool, showed a 42 kDa-band (Fig.5), in agreement with the value calculated from the DNA sequence. These data indicate that the expression product of gene b3 contained B cells epitopes recognized by tuberculous human sera. The species-specificity of these epitopes was tested as for the ORF1 product. Persistence of positive signal after serum pre-absorption indicates the presence, in the a389 protein, of epitopes specific for *M.tuberculosis* and not shared by other mycobacterial species.

The hydrophilicity pattern of the B3 protein is illustrated on figure 9. According to this profile a predicted antigenic peptide G-17-R was synthesised. Antibodies were raised against this peptide in rabbits according to the method described in example 1.

### V. Development of an a171-based serological assay for tuberculosis.

Large scale preparation of the recombinant a171 polypeptide, coded for by gene b2, was based on the expression of this protein in *E.coli* under the form of inclusion bodies. The corresponding cellular fraction was separated from the crude lysate by centrifugation and solubilized. The results of an electrophoretic analysis of the partly purified fraction is shown in Fig. 6. This recombinant product was used to coat microtiter plates. Reference plates were coated with the A60 antigen, the natural mycobacterial antigen complex containing protein B2, in addition to several other proteins. Antigen A60 was prepared according to Cocito and Vanlinden (1986). A series of sera from healthy subjects (non vaccinated and BCG-vaccinated), and patients (56 persons with different forms of tuberculosis) were tested. Data displayed in Fig.7 indicate that most tuberculous sera positive with the A60-ELISA were also positive with the a171-ELISA, which thus represents a species-specific serological assay for tuberculosis.

Based on the production of a B2 protein segment as inclusion bodies in recombinant *E.coli*, the a171 polypeptide corresponding to the carboxyl-terminal portion of B2 has been purified and used as reagent for a serological assay (a171-ELISA) for tuberculosis. According to this preliminary trial, the test was negative for healthy subjects and positive for tuberculosis patients. Although, a larger clinical work, extended to all forms of tuberculosis, is needed to assess the medical importance of this assay, our data suggest that the a171-ELISA, a test endowed with species-specificity, is a promising tool allowing the etiological diagnosis of tuberculosis. Indeed, most of the available immunoassays, including the A60 ELISA previously developed (Baelden et al., 1990; Cocito, 1991), are unable to distinguish tuberculosis from the other mycobacterioses. Yet, such a distinction is important, especially since the prognosis and therapy of mycobacterial diseases are different depending on the mycobacterial species causing the disease.

### LITERATURE REFERENCES

Baelden, M. C., B. Vanderelst, M. Dieng, J. Prignot, and C. Cocito. 1990. Serological analysis of human tuberculosis by an ELISA with mycobacterial antigen 60. Scand J. Infect. Dis. **22:**63-73.
Benoit, C., A. Beschin, M. Desmecht, P. Dekeyser, and C. Cocito. 1989. Delayed hypersensitivity reactions by the mycobacterial antigen A60 and cutaneous testing in tuberculosis. Med. Microbiol. Immunol. **178:**105-112.
Berthet, F. X., J. Rauzier, F. M. Lim, W. Philipp, B. Gicquel, D. Portnoi. 1995. Characterization of the *Mycobacterium tuberculosis* erp gene encoding a potential cell surface protein with repetitive structures. Microbiology. **141:**2123-2130.
Bigi, F., A. Alito, J. C. Fisanotti, M. I. Romano, and A. Cataldi. 1995. Characterization of a novel Mycobacterium bovis secreted antigen containing PGLTS repeats. Infect. Immun. **63:**2581-2586.
Boom, W. H. , K. N. Balaji, R. Nayak, K. Tsukaguchi, and K. A. Chervenak. 1994. Characterization of a 10- to 14-kilodaltons protease-sensitive *Mycobacterium tuberculosis* H37Ra antigen that stimulates human gamma delta T cells. Infect. Immun. **62:**5511-5518.
Borremans, M., L. De Wit, G. Volckaert, J. Ooms, J. De Bruyn, K. Huygen, J. P. Van Vooren, M. Stelandre, R. Verhofstadt, and J. Content. 1989. Cloning, sequence determination and expression of a 32 kDa protein gene from *Mycobacterium tuberculosis*. Infect. Immun. **57:**3123-3130.
Bothwell, A., G.D. Yancopoulos and F.W. Alt. 1990. Methods of cloning and analysis of eukaryotic genes. Eds. Jones and Bartlett Publishers. Boston, MA.
Braibant, M., L. De Wit, P. Peirs, M. Kalai, J. Ooms, A. Drowart, K. Huygen, J. Content. 1994. Structure of the *Mycobacterium tuberculosis* antigen 88, a protein related to *Escherichia coli* PstA periplasmic phosphate permease subunit. Infect. Immun. **62:**849-854.
Carlucci, S., Beschin, A., Tuoso, L., Amegilo, F., Gandolfo, G.M., Cocito, C., Fiorucci, F., C. Saltini, and E. Piccolella.:1993. Mycobacterial antigen complex A60-specific T-cell repertoire during the course of pulmonary tuberculosis. Infect. Immun. **61:**439-447.
Cocito, C., and F. Vanlinden. 1986. Preparation and properties of antigen 60 from *Mycobacterium bovis* BCG. Scand. J. Immunol. **24:**591-602.
Cocito, C. 1991. Properties of the mycobacterial antigen complex A60 and its applications to diagnosis and prognosis of tuberculosis. CHEST. **100:**1687-1693.
Cocito, C., M. Coene, M. De Kesel, P. Gilot, P. Poupart, and P. Vannuffel.:1994. Paratuberculosis. Clin. Microbiol. Rev. **7:**328-345.
Cocito, C., and F. Vanlinden. 1995. Composition and immunoreactivity of different fractions and of the A60 complex from *Mycobacterium bovis* BCG. Scand. J. Immunol. **41:**179-187.
Coetsier, C., M. C. Baelden, M. Coene, and C. Cocito. 1994. Immunological analysis of the components of the antigen complex A60 of *Mycobacterium bovis* BCG. Clin. Diagn. Lab. Immunol. **1:**139-144.
Content, J., A. de a Cuvellerie, L. de Wit, V. Vincent-Levy Fébrault, J. Ooms, and J. de Bruyn. 1991. The genes coding for the antigen 85 complex of *M. tuberculosis* and *M. bovis* BCG are members of a gene family: cloning, sequence determination, and genomic organization of the gene coding for antigen 85 C of *M.tuberculosis*. Infect. Immun. **59:**3205-3212.
Dale, J., and A. Patki. 1990. Mycobacterial gene expression and regulation, p. 172-198. In J. McFadden (ed), Molecular biology of the mycobacteria. Surrey University Press, London.
Daniel, T. D., and S. M. Debanne. 1987. The serodiagnosis of tuberculosis and other mycobacterial diseases by enzyme linked immunosorbent assay. Am. Rev. Respir. Dis. **135:**1137-1151.
Daniel, T. D. 1994.Tuberculosis, p. 710-718. *In* Harrison's principles of internal medicine, Mc Graw-Hill, New York.
De Kesel, M., P. Gilot, M. C. Misonne, M. Coene, and C. Cocito. 1993. Cloning and expression of portions of the 34 kDa protein gene of *Mycobacterium paratuberculosis*: its application to serological analysis of Johne's disease. J. Clin. Microbiol. **31:**947-954.
Deshpande, R. G., M. B. Khan, D. A. Bhat, and R. G. Navalkar. 1994. Purification and partial characterization of a novel 66-kDa seroreactive protein of *Mycobacterium tuberculosis* H37Rv. J. Med. Microbiol. **41:**173-178.
Fabre, I., O. L'Homme, M. Bruneteau, G. Michel, and C. Cocito. 1986. Chemical composition of antigen 60 from *Mycobacterium bovis* BCG. Scand. J. Immunol. **24:**591-602.
Friscia, G., H. M. Vordermeier, G. Pasvol, D. P. Harris, C. Moreno, and J. Ivanyi. 1995. Human T cell responses to peptide epitopes of the 16-kDa antigen in tuberculosis. Clin. Exp. Immunol. **102:**53-57.
Gilot, P., M. De Kesel, M. Coene, and C. Cocito. 1992. Induction of cellular immune reactions by A36, an antigen complex of *Mycobacterium paratuberculosis*. Comparison of A36 and johnin components. Scand. J. Immunol. **36:**811-821.
Gilot, P., M. De Kesel, L. Machtelinckx, C. Coene, and C. Cocito. 1993. Isolation and sequencing of the gene coding for an antigenic 34 kDa protein of *Mycobacterium paratuberculosis*. J. Bacteriol. **175:**4930-4935.
Harris, D. P., H. M. Vordermeier, S. J. Brett, G. Pasvol, C. Moreno, and J. Ivanyi. 1994. Epitope specificity and isoforms of the mycobacterial 19-kilodaltons antigen. Infect. Immun. **62:**2963-2972.
Hawley, D., and W. McClure. 1983. Compilation and analysis of *Escherichia coli* promoter DNA sequences. Nucl. Acids. Res. **11:**2237-2255.
Hermans, P. W., F. Abebe, V. I. Kuteyi, A. H. Kolk, J. E. Thole, and M. Harboe. 1995. Molecular and immunological characterization of the highly conserved antigen 84 from *Mycobacterium tuberculosis* and *Mycobacterium leprae*. Infect. Immun. **63:**954-960.
Hewinson, R. G., S. L. Michell, W. P. Russell, R. A. McAdam, and W. R. Jacobs. 1996. Molecular characterization of MPT83: a seroreactive antigen of *Mycobacterium tuberculosis* with homology to MPT70. Scand. J. Immunol. **43:**490-499.
Holmes, D. S., and M. Quigley. 1981. A rapid boiling method for the preparation of bacterial plasmids. Anal. Biochem. **114:**193-197.
Janin 1979. Nature (London), 277: 491-492.
Karplus P.A. and Schultz G.E. 1985. Naturwissenschaften 72: 212-213.
Laal, S., K. M. Samanich, M. G. Sonnenberg, J. T. Belisle, J. O'Leary, M. S. Simberkoff, S. Zolla-Pazner. 1997. Surrogate marker of preclinical tuberculosis in human immunodeficiency virus infection: antibodies to an 88-kDa secreted antigen of *Mycobacterium tuberculosis*. J. Infect. Dis. **176:**133-143.
Laqueyrerie, A., P. Militzer, F. Romain, K. Eiglmeier, S. Cole, and G. Marchal. 1995. Cloning, sequencing, and expression of the apa gene coding for the *Mycobacterium tuberculosis* 45/47-kilodalton secreted antigen complex. Infect. Immun. **63:**4003-4010.
Manca, C., K. Lyashchenko, H. G. Wiker, D. Usai, R. Colangeli, and M. L. Gennaro. 1997. Molecular cloning, purification, and serological characterization of MPT63, a novel antigen secreted by *Mycobaterium tuberculosis*. Infect. Immun. **65:**16-23.
Matsumoto, S., T. Matsuo, N. Ohara, H. Hotokezaka, M. Naito, J. Minami, and T. Yamada. 1995. Cloning and sequencing of a unique antigen MPT70 from *Mycobacterium tuberculosis* H37Rv and expression in BCG using E. coli-mycobacteria shuttle vector. Scand. J. Immunol. **41:**281-287.
O'Loan, C. J., J. M. Pollock, J. Hanna, and S. D. Neill. 1994. Immunoblot analysis of humoral immune responses to *Mycobacterium bovis* in expermentally infected cattle: early recognition of a 26-kilodaltons antigen. Clin. Diagn. Lab. Immunol. **1:**608-611.
Parker J.M., Guo D, and Hodges RS. 1986. Biochemistry 25: 5425-5432.
Pollock, J. M., and P. Andersen. 1997. The potential of the ESAT-6 antigen secreted by virulent mycobacteria for specific diagnosis of tuberculosis. J. Infect. Dis. **175:**1251-1254.
Prestidge, R. L., P. M. Grandison, D. W. Chuk, R. J. Booth, and J. D. Watson. 1995. Production of the 19-kDa antigen of *Mycobacterium tuberculosis* in *Escherichia coli* and its purification. Gene. **164:**129-132.
Romain, F., A. Laqueyrerie, P. Militzer, P. Pescher, P. Chavarot, M. Lagranderie, G. Auregan, M. Gheorghiu, and G. Marchal. 1993. Identification of a *Mycobacterium bovis* BCG 45/47-kilodaltons antigen complex, an immunodominant target for antibody response after immunization with living bacteria. Infect. Immun. **61:**742-750.
Sambrook, J., E. F. Fritsch, and T. Maniatis. 1989. Molecular cloning: a laboratory manual. Cold Spring Harbor Laboratory, Cold Spring Harbor, N. Y.
Sinha, R. K., I. Verma, and G. K. Khuller. 1997. Immunobiological properties of a 30 kDa secretory protein of *Mycobacterium tuberculosis* H37Ra. Vaccine. **15:**689-699.
Singh, M., A. B. Andersen, J. E. McCarthy, R. Rohde, H. Schutte, E. Sanders, and K. N. Timmis. 1992. The *mycobacterium tuberculosis* 38-kDa antigen: overproduction in *Escherichia coli*, purification and characterization. Gene. **117:**53-60.
Sorensen, A. L., S. Nagai, G. Houen, P. Andersen, and A. B. Andersen. 1995. Purification and characterization of a low-molecular-mass T-cell antigen secreted by *Mycobaterium tuberculosis*. Infect. Immun. **63:**1710-1717.
Ulmer J, Donnelly J, Parker S, et al. 1993. Heterologous protection against influenza by injection of DNA encoding a viral protein. Science, 259: 1745-1749.
Verbon, A., R. A. Hartskeel, C. Moreno, and A. H. Kolk. 1992. Characterization of B cell epitopes on the 16K antigen of *Mycobacterium tuberculosis*. Clin. Exp. Immunol. **89:**395-401.
Youmans, G. P. 1985. The biological and clinicalm basis of Infections Diseases, p.348-367. In Youmans, G. P., P. Y. Paterson, H. M. Sommers (eds), Saunders, Philadelphia.
Young, D. B., S. H. E. Kaufmann, P. W. M. Hermans, and J. E. R. Thole. 1992. Mycobacterial protein antigens: a compilation. Mol. Microbiol. **6:**133-145.
Young, D., J. Ivanyi, J. Cox, and J. Lamp. 1987. The 65 kDa antigen of mycobacteria - a common bacterial protein. Immunol. Today. **8:**215-219.
Zou, Y. L., M. P. Van Antwerpen, G. Q. Shi, Q. X. Chen, C. J. M. Sindic, and C. Cocito. 1994a. Analysis of tuberculous meningitis cases by an immunoblotting assay based on a mycobacterial antigen complex. Clin. Diagn. Labor. Immunology. **1:**353-356.
Zou, Y. L., J. D. Zhang, M. H. Chen, G. Q. Shi, and C. Cocito. 1994b. Serological analysis of pulmonary and extrapulmonary tuberculosis with ELISA assays for different immunoglobulins. Clin. Inf. Dis. **19:**1084-1091.

## Claims

1. An isolated nucleic acid of *M. tuberculosis* containing a nucleotide sequence with a maximum length of 3000 contiguous nucleotides, wherein said nucleotide sequence contains at least two overlapping ORF's which encode at least a fragment of the following two antigenic proteins recognized by a pool of sera from tuberculous patients:
(i) a B2 protein represented by SEQ ID N0 2, or a protein homologous to B2 containing in its amino acid sequence at least one conservative amino acid substitution (according to Table 1) with respect to the sequence represented by SEQ ID N0 2, and
(ii) a B3 protein represented by SEQ ID N0 3, or a protein homologous to B3 containing in its amino acid sequence at least one conservative amino acid substitution (according to Table 1) with respect to the sequence represented by SEQ ID N0 3,
or a fragment of said nucleic acid, said fragment containing at least 15, preferably at least 30 and more preferably at least 45 contiguous nucleotides of the sequence of said nucleic acid, and encoding at least one epitope of the B2 and/or B3 protein, or its homologues.

2. An isolated nucleic acid according to claim 1, wherein said nucleotide sequence is selected from:
(i) the nucleotide sequence as represented in figure 2 (SEQ ID N0 1),
(ii) a nucleotide sequence which hybridizes to the nucleotide sequence of (i) and which encodes a B2 protein, or a protein homologous to B2, and/or a B3 protein, or a protein homologous to B3, with said B2 (B3) protein and its homologues as defined in claim 1,
(iii) a nucleotide sequence which is degenerate as a result of the genetic code to any of the nucleotide sequences as defined in (i) and (ii) and which encodes a B2 protein, or a protein homologous to B2, and/or a B3 protein, or a protein homologous to B3, with said B2 (B3) protein and its homologues as defined in claim 1, and
(iv) a fragment of any of the nucleotide sequences as defined in (i) to (iii), said fragment containing a stretch of at least 15, preferably 30 and more preferably 45 contiguous nucleotides of any of the nucleotide sequences as defined in (i) to (iii), and encoding at least one epitope of the B2 and/or B3 protein, or the homologous proteins thereof.

3. An isolated nucleic acid according to any of claims 1 to 2, wherein said nucleotide sequence extends from position (x) to position (y) in the sequence of figure 2 (SEQ ID N0 1), with (x) and (y) representing any of the following values:
- from position (71) to position (1951),
- from position (398) to position (1951),
- from position (1063) to position (2604),
- from position (272) to position (1438),
- from position (1439) to position (1951),
- from position (1438) to position (2604),
or a fragment of said nucleic acid, said fragment containing at least 15, preferably at least 30 and more preferably at least 45 contiguous nucleotides of the sequence of said nucleic acid, and encoding at least one epitope of the B2 and/or B3 protein.

4. A recombinant vector, for cloning and/or expression purposes, into which a nucleic acid according to any of claims 1 to 3, or a fragment of said nucleic acid, has been inserted and wherein, in case of an expression vector, the coding sequence of said nucleic acid is operably linked to an expression cassette enabling the expression of the coding sequence by a host cell.

5. A host cell transformed by a recombinant vector according to claim 4, with said host cell being preferably a prokaryotic organism, preferably chosen from among the following group of organisms: *E. coli*, *Streptomyces* species, *Corynebacterium* species, such as e.g. *C. glutamicum*, or fast-growing *Mycobacterium* species, such as e.g. *M. smegmatis*.

6. A polypeptide encoded by any of the nucleic acids according to claims 1 to 3.

7. A polypeptide according to claim 6, having at least one of the following amino acid sequences:
(i) the amino acid sequence of the B2 protein represented by SEQ ID N0 2,
(ii) the amino acid sequence of the B3 protein represented by SEQ ID N0 3,
(iii) the amino acid sequence extending from position (110) to position (626) in SEQ ID N0 2,
(iv) the amino acid sequence extending from position (68) to position (456) in SEQ ID N0 2,
(v) the amino acid sequence extending from position (457) to position (626) in SEQ ID N0 2,
(vi) the amino acid sequence extending from position (126) to position (513) in SEQ ID N0 3,
or a fragment of any of the polypeptides according to (i) to (vi), said fragment consisting of a stretch of at least 6, preferablyl 7, 8, 9, 10, 11, 12, 13, 14, 15, 16, 17, 18, 19, 20, 21, 22, 23, 24, 25, 30, 50 or more contiguous amino acids of the sequence represented by SEQ ID N0 2 or SEQ ID N0 3, and being recognized by a pool of sera from tuberculous patients.

8. A fusion protein comprising an amino acid sequence of a polypeptide according to any of claims 6 to 7, or a fragment thereof, linked to a heterologous amino acid sequence.

9. A method for the production of a recombinant polypeptide according to any of claims 6 to 7, or a fusion protein according to claim 8, comprising at least the following steps:
- culturing a transformed cellular host according to claim 5 under conditions allowing the heterologous expression of the polypeptide or the fusion protein which is encoded,
- isolating the polypeptide or fusion protein from the culture broth.

10. An antibody raised against a polypeptide according to any of claims 6 to 7, or raised against a fusion protein according to claim 8, more preferably a monoclonal antibody.

11. A method for the *in vitro* detection of tuberculosis disease in a mammal subject, based on the detection of anti- *M. tuberculosis* antibodies present in a sample taken from said subject, comprising the steps of
- contacting said sample with a polypeptide according to any of claims 6 to 7, or with a fusion protein according to claim 8, under conditions allowing the formation of an immunological complex, and
- detecting the presence of an immunological complex formed between said polypeptide and the anti - *M. tuberculosis* antibodies which may be present in said sample.

12. A method for the *in vitro* detection of tuberculosis disease in a mammal subject, based on the detection of *M. tuberculosis* antigens present in a sample taken from said subject, and comprising the steps of
- if necessary, pretreatment of said sample in order to release the *M. tuberculosis* antigens which may be present in said sample,
- contacting said sample with an antibody according to claim 10, under conditions allowing the formation of an immunological complex, and
- detecting the presence of an immunological complex formed between said antibody and the *M. tuberculosis* antigens which may be present in said sample.

13. An immunodiagnostic kit for the *in vitro* detection of tuberculosis disease in mammals, said kit comprising a polypeptide according to any of claims 6 to 7, or a fusion protein according to claim 8, or an antibody according to claim 10.

14. A vaccine composition for the prevention of tuberculosis disease in mammals, said vaccine composition comprising as an active principle a polypeptide according to any of claims 6 to 7, or a fusion protein according to claim 8, or a nucleic acid according to any of claims 1 to 3, or a recombinant vector according to claim 4, said active principle being combined with a pharmaceutically acceptable carrier.


SEQUENCE LISTING

(1) GENERAL INFORMATION:

(i) APPLICANT:
(A) NAME: Innogenetics N.V.
(B) STREET: Industriepark Zwijnaarde 7, box 4
(C) CITY: Ghent
(E) COUNTRY: Belgium
(F) POSTAL CODE (ZIP): B-9052
(G) TELEPHONE: 00 32 9 241 07 11
(H) TELEFAX: 00 32 9 241 07 99

(ii) TITLE OF INVENTION: New polypeptides and nucleic acids encoding
the same for the diagnosis and prevention of tuberculosis
disease.

(iii) NUMBER OF SEQUENCES: 9

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98870127.2

(2) INFORMATION FOR SEQ ID NO: 1:

(i) SEQUENCE CHARACTERISTICS:
(A) LENGTH: 2708 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(ix) FEATURE:
(A) NAME/KEY: CDS
(B) LOCATION:71..1951

(ix) FEATURE:
(A) NAME/KEY: mat_peptide
(B) LOCATION:71..1948

(ix) FEATURE:
(A) NAME/KEY: CDS
(B) LOCATION:1063..2599

(ix) FEATURE:
(A) NAME/KEY: mat_peptide
(B) LOCATION:1063..2596

(xi) SEQUENCE DESCRIPTION: SEQ ID NO: 1:

GACTGCAAAG CTGGGTTGTC CCGCCGACAC CGCATTCGCG CGCGTCGCTG GCCCGATACG 60

AGATAGCGCA ATG ACC ACC CCT TCC CAC CTG TCC GAC CGC TAC GAA CTT 109

Met Thr Thr Pro Ser His Leu Ser Asp Arg Tyr Glu Leu
1               5                   10

GGC GAA ATC CTT GGA TTT GGG GGC ATG TCC GAG GTC CAC CTG GCC CGC     157
Gly Glu Ile Leu Gly Phe Gly Gly Met Ser Glu Val His Leu Ala Arg
    15                  20                  25

GAC CTC CGG TTG CAC CGC GAC GTT GCG GTC AAG GTG CTG CGC GCT GAT     205
Asp Leu Arg Leu His Arg Asp Val Ala Val Lys Val Leu Arg Ala Asp
30                  35                  40                  45

CTA GCC CGC GAT CCC AGT TTT TAC CTT CGC TTC CGG CGT GAG GCG CAA     253
Leu Ala Arg Asp Pro Ser Phe Tyr Leu Arg Phe Arg Arg Glu Ala Gln
                50                  55                  60

AAC GCC GCG GCA TTG AAC CAC CCT GCA ATC GTC GCG GTC TAC GAC ACC     301
Asn Ala Ala Ala Leu Asn His Pro Ala Ile Val Ala Val Tyr Asp Thr
            65                  70                  75

GGT GAA GCC GAA ACG CCC GCC GGG CCA TTG CCC TAC ATC GTC ATG GAA     349
Gly Glu Ala Glu Thr Pro Ala Gly Pro Leu Pro Tyr Ile Val Met Glu
        80                  85                  90

TAC GTC GAC GGC GTT ACC CTG CGC GAC ATT GTC CAC ACC GAA GGG CCG     397
Tyr Val Asp Gly Val Thr Leu Arg Asp Ile Val His Thr Glu Gly Pro
    95                  100                 105

ATG ACG CCC AAA CGC GCC ATC GAG GTC ATC GCC GAC GCC TGC CAA GCG     445
Met Thr Pro Lys Arg Ala Ile Glu Val Ile Ala Asp Ala Cys Gln Ala
110                 115                 120                 125

CTG AAC TTC AGT CAT CAG AAC GGA ATC ATC CAC CGT GAC GTC AAG CCG     493
Leu Asn Phe Ser His Gln Asn Gly Ile Ile His Arg Asp Val Lys Pro
                130                 135                 140

GCG AAC ATC ATG ATC AGC GCG ACC AAT GCA GTA AAG GTG ATG GAT TTC     541
Ala Asn Ile Met Ile Ser Ala Thr Asn Ala Val Lys Val Met Asp Phe
            145                 150                 155

GGC ATC GCC CGC GCC ATT GCC GAC AGC GGC AAC AGC GTG ACC CAG ACC     589
Gly Ile Ala Arg Ala Ile Ala Asp Ser Gly Asn Ser Val Thr Gln Thr
        160                 165                 170

GCA GCA GTG ATC GGC ACG GCG CAG TAC CTG TCA CCC GAA CAG GCC CGG     637
Ala Ala Val Ile Gly Thr Ala Gln Tyr Leu Ser Pro Glu Gln Ala Arg
    175                 180                 185

GGT GAT TCC GTC GAC GCC CGA TCC GAT GTC TAT TCC TTG GGC TGT GTT     685
Gly Asp Ser Val Asp Ala Arg Ser Asp Val Tyr Ser Leu Gly Cys Val
190                 195                 200                 205

CTT TAT GAA GTC CTC ACC GGG GAG CCA CCT TTC ACC GGC GAC TCA CCC     733
Leu Tyr Glu Val Leu Thr Gly Glu Pro Pro Phe Thr Gly Asp Ser Pro
                210                 215                 220

GTC TCG GTT GCC TAC CAA CAT GTG CGC GAA GAC CCG ATC CCA CCT TCG     781
Val Ser Val Ala Tyr Gln His Val Arg Glu Asp Pro Ile Pro Pro Ser
            225                 230                 235

GCG CGG CAC GAA GGC CTC TCC GCC GAC CTG GAC GCC GTC GTT CTC AAG     829
Ala Arg His Glu Gly Leu Ser Ala Asp Leu Asp Ala Val Val Leu Lys
        240                 245                 250

GCG CTG GCC AAA AAT CCG GAA AAC CGC TAT CAG ACA GCG GCG GAG ATG     877
Ala Leu Ala Lys Asn Pro Glu Asn Arg Tyr Gln Thr Ala Ala Glu Met
    255                 260                 265

CGC GCC GAC CTG GTC CGC GTG CAC AAC GGT GAG CCG CCC GAG GCG CCC 925
Arg Ala Asp Leu Val Arg Val His Asn Gly Glu Pro Pro Glu Ala Pro
270                 275                 280                 285

AAA GTG CTC ACC GAT GCC GAG CGG ACC TCG CTG CTG TCG TCT GCG GCC 973
Lys Val Leu Thr Asp Ala Glu Arg Thr Ser Leu Leu Ser Ser Ala Ala
                290                 295                 300

GGC AAC CTT AGC GGT CCG CGC ACC GAT CCG CTA CCA CGC CAG GAC TTA 1021
Gly Asn Leu Ser Gly Pro Arg Thr Asp Pro Leu Pro Arg Gln Asp Leu
            305                 310                 315

GAC GAC ACC GAC CGT GAC CGC AGC ATC GGT TCG GTG GGC CGT TGG GTT 1069
Asp Asp Thr Asp Arg Asp Arg Ser Ile Gly Ser Val Gly Arg Trp Val
        320                 325                 330

GCG GTG GTC GCC GTG CTC GCT GTG CTG ACC GTC GTG GTA ACC ATC GCC 1117
Ala Val Val Ala Val Leu Ala Val Leu Thr Val Val Val Thr Ile Ala
    335                 340                 345

ATC AAC ACG TTC GGC GGC ATC ACC CGC GAC GTT CAA GTT CCC GAC GTT 1165
Ile Asn Thr Phe Gly Gly Ile Thr Arg Asp Val Gln Val Pro Asp Val
350                 355                 360                 365

CGG GGT CAA TCC TCC GCC GAC GCC ATC GCC ACA CTG CAA AAC CGG GGC 1213
Arg Gly Gln Ser Ser Ala Asp Ala Ile Ala Thr Leu Gln Asn Arg Gly
                370                 375                 380

TTC AAA ATC CGC ACC TTG CAG AAG CCG GAC TCG ACA ATC CCA CCG GAC 1261
Phe Lys Ile Arg Thr Leu Gln Lys Pro Asp Ser Thr Ile Pro Pro Asp
            385                 390                 395

CAC GTT ATC GGC ACC GAC CCG GCC GCC AAC ACG TCG GTG AGT GCA GGC 1309
His Val Ile Gly Thr Asp Pro Ala Ala Asn Thr Ser Val Ser Ala Gly
        400                 405                 410

GAC GAG ATC ACA GTC AAC GTG TCC ACC GGA CCG GAG CAA CGC GAA ATA 1357
Asp Glu Ile Thr Val Asn Val Ser Thr Gly Pro Glu Gln Arg Glu Ile
    415                 420                 425

CCC GAC GTC TCC ACG CTG ACA TAC GCC GAA GCG GTC AAG AAA CTG ACT 1405
Pro Asp Val Ser Thr Leu Thr Tyr Ala Glu Ala Val Lys Lys Leu Thr
430                 435                 440                 445

GCC GCC GGA TTC GGC CGC TTC AAG CAA GCG AAT TCG CCG TCC ACC CCG 1453
Ala Ala Gly Phe Gly Arg Phe Lys Gln Ala Asn Ser Pro Ser Thr Pro
                450                 455                 460

GAA CTG GTG GGC AAG GTC ATC GGG ACC AAC CCG CCA GCC AAC CAG ACG 1501
Glu Leu Val Gly Lys Val Ile Gly Thr Asn Pro Pro Ala Asn Gln Thr
            465                 470                 475

TCG GCC ATC ACC AAT GTG GTC ATC ATC ATC GTT GGC TCT GGT CCG GCG 1549
Ser Ala Ile Thr Asn Val Val Ile Ile Ile Val Gly Ser Gly Pro Ala
        480                 485                 490

ACC AAA GAC ATT CCC GAT GTC GCG GGC CAG ACC GTC GAC GTG GCG CAG 1597
Thr Lys Asp Ile Pro Asp Val Ala Gly Gln Thr Val Asp Val Ala Gln
    495                 500                 505

AAG AAC CTC AAC GTC TAC GGC TTC ACC AAA TTC AGT CAG GCC TCG GTG 1645
Lys Asn Leu Asn Val Tyr Gly Phe Thr Lys Phe Ser Gln Ala Ser Val
510                 515                 520                 525

GAC AGC CCC CGT CCC GCC GGC GAG GTG ACC GGC ACC AAT CCA CCC GCA 1693
Asp Ser Pro Arg Pro Ala Gly Glu Val Thr Gly Thr Asn Pro Pro Ala

                530                 535                 540

GGC ACC ACA GTT CCG GTC GAT TCA GTC ATC GAA CTA CAG GTG TCC AAG 1741
Gly Thr Thr Val Pro Val Asp Ser Val Ile Glu Leu Gln Val Ser Lys
            545                 550                 555

GGC AAC CAA TTC GTC ATG CCC GAC CTA TCC GGC ATG TTC TGG GTC GAC 1789
Gly Asn Gln Phe Val Met Pro Asp Leu Ser Gly Met Phe Trp Val Asp
        560                 565                 570

GCC GAA CCA CGA TTG CGC GCG CTG GGC TGG ACC GGG ATG CTC GAC AAA 1837
Ala Glu Pro Arg Leu Arg Ala Leu Gly Trp Thr Gly Met Leu Asp Lys
    575                 580                 585

GGG GCC GAC GTC GAC GCC GGT GGC TCC CAA CAC AAC CGG GTC GTC TAT 1885
Gly Ala Asp Val Asp Ala Gly Gly Ser Gln His Asn Arg Val Val Tyr
590                 595                 600                 605

CAA AAC CCG CCG GCG GGG ACC GGC GTC AAC CGG GAC GGC ATC ATC ACG 1933
Gln Asn Pro Pro Ala Gly Thr Gly Val Asn Arg Asp Gly Ile Ile Thr
                610                 615                 620

CTG AGG TTC GGC CAG TAG CTTCGCCCGC GCTAGCGGTT GAAGTTGGGA 1981
Leu Arg Phe Gly Gln *
            625

AGTCCGGTGA GATGGCGGTG AGCACTTCGT TTTCCAGCCG ACGTACCAGG GTGTCGTCTT 2041

GCGTCCATCC GCAGCAGGTG AGCCAGTTGG CCAGTATGCG GTGCCCGCCC TCGGTGAGAA 2101

TCGACTCCGG ATGGAACTGG ACACCGTGGA TCGGCAGCCC GGTGTGCTGC ACGGCCATGA 2161

TCACACCGCT GCTAGTGCGG GCCGTGACCC TCAGCACCGC TGGCAGCGAC TTAGGCAGAA 2221

TTTTCAACGA ATGGTATCGA GTGGCCGTGA AGGGATCCGG AAGCCCTTGT AGCACACCGA 2281

CATTGGTGTG GAATACGCTG CTGGTCTTGC CGTGCAATAG CTCGGGCGCA CGGTCCACGG 2341

TGGCGCCGAA CGCAACGCCG ATGGCTTGGT GCCCAAGGCA GACCCCCAGC AAAGGGGTGT 2401

GTGCTGCCGC ACACGCGTGC ACGATACTCA CCGACGCGCC CGCGCGCTCC GGGGTACCCG 2461

GACCGGGACT GAGCAGGACA CCGTCGAATT GGCCGGCGAC GGCGGCCTCA TCGGATAACC 2521

GGTGGTCGTC GTTGCGCCAC ACCTCGGCCT CGATGCCGAG CTGGCCGAGG TACTGCACCA 2581

GGTTGAACAC GAAGCTGTCG TAGTTGTCAA CGACCAGGAT CCGCATCGTG ACAGGCTACC 2641

GCCTGACCCT GCCGGCAGTC GTTCAGTAGT CCAAGGGGCC TGCAGGCTGC GCGAAGTGCA 2701

TCCGGAC 2708

(2) INFORMATION FOR SEQ ID NO: 2:

(i) SEQUENCE CHARACTERISTICS:
(A) LENGTH: 627 amino acids
(B) TYPE: amino acid
(D) TOPOLOGY: linear

(ii) MOLECULE TYPE: protein
(xi) SEQUENCE DESCRIPTION: SEQ ID NO: 2:

Met Thr Thr Pro Ser His Leu Ser Asp Arg Tyr Glu Leu Gly Glu Ile
1               5                   10                  15

Leu Gly Phe Gly Gly Met Ser Glu Val His Leu Ala Arg Asp Leu Arg
            20                  25                  30

Leu His Arg Asp Val Ala Val Lys Val Leu Arg Ala Asp Leu Ala Arg
        35                  40                  45

Asp Pro Ser Phe Tyr Leu Arg Phe Arg Arg Glu Ala Gln Asn Ala Ala
    50                  55                  60

Ala Leu Asn His Pro Ala Ile Val Ala Val Tyr Asp Thr Gly Glu Ala
65                  70                  75                  80

Glu Thr Pro Ala Gly Pro Leu Pro Tyr Ile Val Met Glu Tyr Val Asp
                85                  90                  95

Gly Val Thr Leu Arg Asp Ile Val His Thr Glu Gly Pro Met Thr Pro
            100                 105                 110

Lys Arg Ala Ile Glu Val Ile Ala Asp Ala Cys Gln Ala Leu Asn Phe
        115                 120                 125

Ser His Gln Asn Gly Ile Ile His Arg Asp Val Lys Pro Ala Asn Ile
    130                 135                 140

Met Ile Ser Ala Thr Asn Ala Val Lys Val Met Asp Phe Gly Ile Ala
145                 150                 155                 160

Arg Ala Ile Ala Asp Ser Gly Asn Ser Val Thr Gln Thr Ala Ala Val
                165                 170                 175

Ile Gly Thr Ala Gln Tyr Leu Ser Pro Glu Gln Ala Arg Gly Asp Ser
            180                 185                 190

Val Asp Ala Arg Ser Asp Val Tyr Ser Leu Gly Cys Val Leu Tyr Glu
        195                 200                 205

Val Leu Thr Gly Glu Pro Pro Phe Thr Gly Asp Ser Pro Val Ser Val
    210                 215                 220

Ala Tyr Gln His Val Arg Glu Asp Pro Ile Pro Pro Ser Ala Arg His
225                 230                 235                 240

Glu Gly Leu Ser Ala Asp Leu Asp Ala Val Val Leu Lys Ala Leu Ala
                245                 250                 255

Lys Asn Pro Glu Asn Arg Tyr Gln Thr Ala Ala Glu Met Arg Ala Asp
            260                 265                 270

Leu Val Arg Val His Asn Gly Glu Pro Pro Glu Ala Pro Lys Val Leu
        275                 280                 285

Thr Asp Ala Glu Arg Thr Ser Leu Leu Ser Ser Ala Ala Gly Asn Leu
    290                 295                 300

Ser Gly Pro Arg Thr Asp Pro Leu Pro Arg Gln Asp Leu Asp Asp Thr
305                 310                 315                 320

Asp Arg Asp Arg Ser Ile Gly Ser Val Gly Arg Trp Val Ala Val Val
                325                 330                 335

Ala Val Leu Ala Val Leu Thr Val Val Val Thr Ile Ala Ile Asn Thr
            340                 345                 350

Phe Gly Gly Ile Thr Arg Asp Val Gln Val Pro Asp Val Arg Gly Gln
        355                 360                 365

Ser Ser Ala Asp Ala Ile Ala Thr Leu Gln Asn Arg Gly Phe Lys Ile
    370                 375                 380

Arg Thr Leu Gln Lys Pro Asp Ser Thr Ile Pro Pro Asp His Val Ile
385                 390                 395                 400

Gly Thr Asp Pro Ala Ala Asn Thr Ser Val Ser Ala Gly Asp Glu Ile
                405                 410                 415

Thr Val Asn Val Ser Thr Gly Pro Glu Gln Arg Glu Ile Pro Asp Val
            420                 425                 430

Ser Thr Leu Thr Tyr Ala Glu Ala Val Lys Lys Leu Thr Ala Ala Gly
        435                 440                 445

Phe Gly Arg Phe Lys Gln Ala Asn Ser Pro Ser Thr Pro Glu Leu Val
    450                 455                 460

Gly Lys Val Ile Gly Thr Asn Pro Pro Ala Asn Gln Thr Ser Ala Ile
465                 470                 475                 480

Thr Asn Val Val Ile Ile Ile Val Gly Ser Gly Pro Ala Thr Lys Asp
                485                 490                 495

Ile Pro Asp Val Ala Gly Gln Thr Val Asp Val Ala Gln Lys Asn Leu
            500                 505                 510

Asn Val Tyr Gly Phe Thr Lys Phe Ser Gln Ala Ser Val Asp Ser Pro
        515                 520                 525

Arg Pro Ala Gly Glu Val Thr Gly Thr Asn Pro Pro Ala Gly Thr Thr
    530                 535                 540

Val Pro Val Asp Ser Val Ile Glu Leu Gln Val Ser Lys Gly Asn Gln
545                 550                 555                 560

Phe Val Met Pro Asp Leu Ser Gly Met Phe Trp Val Asp Ala Glu Pro
                565                 570                 575

Arg Leu Arg Ala Leu Gly Trp Thr Gly Met Leu Asp Lys Gly Ala Asp
            580                 585                 590

Val Asp Ala Gly Gly Ser Gln His Asn Arg Val Val Tyr Gln Asn Pro
        595                 600                 605

Pro Ala Gly Thr Gly Val Asn Arg Asp Gly Ile Ile Thr Leu Arg Phe
    610                 615                 620

Gly Gln  *
625

(2) INFORMATION FOR SEQ ID NO: 3:

     (i) SEQUENCE CHARACTERISTICS:
          (A) LENGTH: 513 amino acids
          (B) TYPE: amino acid
          (D) TOPOLOGY: linear

    (ii) MOLECULE TYPE: protein

    (xi) SEQUENCE DESCRIPTION: SEQ ID NO: 3:

     Leu Gly Cys Gly Gly Arg Arg Ala Arg Cys Ala Asp Arg Arg Gly Asn
     1               5                   10                  15

His Arg His Gln His Val Arg Arg His His Pro Arg Arg Ser Ser Ser
            20                  25                  30

Arg Arg Ser Gly Ser Ile Leu Arg Arg Arg His Arg His Thr Ala Lys
        35                  40                  45

Pro Gly Leu Gln Asn Pro His Leu Ala Glu Ala Gly Leu Asp Asn Pro
    50                  55                  60

Thr Gly Pro Arg Tyr Arg His Arg Pro Gly Arg Gln His Val Gly Glu
65                  70                  75                  80

Cys Arg Arg Arg Asp His Ser Gln Arg Val His Arg Thr Gly Ala Thr
                85                  90                  95

Arg Asn Thr Arg Arg Leu His Ala Asp Ile Arg Arg Ser Gly Gln Glu
            100                 105                 110

Thr Asp Cys Arg Arg Ile Arg Pro Leu Gln Ala Ser Glu Phe Ala Val
        115                 120                 125

His Pro Gly Thr Gly Gly Gln Gly His Arg Asp Gln Pro Ala Ser Gln
    130                 135                 140

Pro Asp Val Gly His His Gln Cys Gly His His His Arg Trp Leu Trp
145                 150                 155                 160

Ser Gly Asp Gln Arg His Ser Arg Cys Arg Gly Pro Asp Arg Arg Arg
                165                 170                 175

Gly Ala Glu Glu Pro Gln Arg Leu Arg Leu His Gln Ile Gln Ser Gly
            180                 185                 190

Leu Gly Gly Gln Pro Pro Ser Arg Arg Arg Gly Asp Arg His Gln Ser
        195                 200                 205

Thr Arg Arg His His Ser Ser Gly Arg Phe Ser His Arg Thr Thr Gly
    210                 215                 220

Val Gln Gly Gln Pro Ile Arg His Ala Arg Pro Ile Arg His Val Leu
225                 230                 235                 240

Gly Arg Arg Arg Thr Thr Ile Ala Arg Ala Gly Leu Asp Arg Asp Ala
                245                 250                 255

Arg Gln Arg Gly Arg Arg Arg Arg Arg Trp Leu Pro Thr Gln Pro Gly
            260                 265                 270

Arg Leu Ser Lys Pro Ala Gly Gly Asp Arg Arg Gln Pro Gly Arg His
        275                 280                 285

His His Ala Glu Val Arg Pro Val Ala Ser Pro Ala Leu Ala Val Glu
    290                 295                 300

Val Gly Lys Ser Gly Glu Met Ala Val Ser Thr Ser Phe Ser Ser Arg
305                 310                 315                 320

Arg Thr Arg Val Ser Ser Cys Val His Pro Gln Gln Val Ser Gln Leu
                325                 330                 335

Ala Ser Met Arg Cys Pro Pro Ser Val Arg Ile Asp Ser Gly Trp Asn
            340                 345                 350

Trp Thr Pro Trp Ile Gly Ser Pro Val Cys Cys Thr Ala Met Ile Thr
        355                 360                 365

Pro Leu Leu Val Arg Ala Val Thr Leu Ser Thr Ala Gly Ser Asp Leu
    370                 375                 380

Gly Arg Ile Phe Asn Glu Trp Tyr Arg Val Ala Val Lys Gly Ser Gly
385                 390                 395                 400

Ser Pro Cys Ser Thr Pro Thr Leu Val Trp Asn Thr Leu Leu Val Leu
                405                 410                 415

Pro Cys Asn Ser Ser Gly Ala Arg Ser Thr Val Ala Pro Asn Ala Thr
            420                 425                 430

Pro Met Ala Trp Cys Pro Arg Gln Thr Pro Ser Lys Gly Val Cys Ala
        435                 440                 445

Ala Ala His Ala Cys Thr Ile Leu Thr Asp Ala Pro Ala Arg Ser Gly
    450                 455                 460

Val Pro Gly Pro Gly Leu Ser Arg Thr Pro Ser Asn Trp Pro Ala Thr
465                 470                 475                 480

Ala Ala Ser Ser Asp Asn Arg Trp Ser Ser Leu Arg His Thr Ser Ala
                485                 490                 495

Ser Met Pro Ser Trp Pro Arg Tyr Cys Thr Arg Leu Asn Thr Lys Leu
            500                 505                 510

Ser

(2) INFORMATION FOR SEQ ID NO: 4:

     (i) SEQUENCE CHARACTERISTICS:
          (A) LENGTH: 14 amino acids
          (B) TYPE: amino acid
          (C) STRANDEDNESS:
          (D) TOPOLOGY: linear

    (ii) MOLECULE TYPE: peptide

   (iii) HYPOTHETICAL: NO

    (iv) ANTI-SENSE: NO

    (xi) SEQUENCE DESCRIPTION: SEQ ID NO: 4:

Lys Gly Ala Asp Val Asp Ala Gly Gly Ser Gln His Asn Arg
1               5                   10

(2) INFORMATION FOR SEQ ID NO: 5:

     (i) SEQUENCE CHARACTERISTICS:
          (A) LENGTH: 17 amino acids
          (B) TYPE: amino acid
          (C) STRANDEDNESS:
          (D) TOPOLOGY: linear

    (ii) MOLECULE TYPE: peptide

   (iii) HYPOTHETICAL: NO

    (iv) ANTI-SENSE: NO

(xi) SEQUENCE DESCRIPTION: SEQ ID NO: 5:

Gly Gln Pro Pro Ser Arg Arg Arg Gly Asp Arg His Gln Ser Thr Arg
1               5                   10                  15

Arg

(2) INFORMATION FOR SEQ ID NO: 6:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO: 6:

GCGGGCGTT TCGGCTTCAC C                                                21

(2) INFORMATION FOR SEQ ID NO: 7:

(i) SEQUENCE CHARACTERISTICS:
(A) LENGTH: 132 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO: 7:

CAGGGAATTC GAGCTCGGTA CCCGGGGAGG CCTGCACGTG ATCGAGGGCA GACACCATCA        60

CCACCATCAC TAATAGTTAA TTAACTGCAG GGATCCTCTA GAGTCGACCG GCATGCAAGC       120

TTAAGTAAGT AA                                                         132

(2) INFORMATION FOR SEQ ID NO: 8:

(i) SEQUENCE CHARACTERISTICS:
(A) LENGTH: 133 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO: 8:

CAGCCGAATT CGAGCTCGGT ACCCGGGGAG GCCTGCACGT GATCGAGGGC AGACACCATC 60

ACCACCATCA CTAATAGTTA ATTAACTGCA GGGATCCTCT AGAGTCGACC GGCATGCAAG 120

CTTAAGTAAG TAA 133

(2) INFORMATION FOR SEQ ID NO: 9:

(i) SEQUENCE CHARACTERISTICS:
(A) LENGTH: 134 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO: 9:

CAGCCCGAAT TCGAGCTCGG TACCCGGGGA GGCCTGCACG TGATCGAGGG CAGACACCAT 60

CACCACCATC ACTAATAGTT AATTAACTGC AGGGATCCTC TAGAGTCGAC CGGCATGCAA 120

GCTTAAGTAA GTAA 134